(19) Europäisches Patentamt
European Patent Office
Office européen des brevets

(11) **EP 3 565 530 B1**

(12) **EUROPEAN PATENT SPECIFICATION**

(45) Date of publication and mention of the grant of the patent:
**19.08.2026 Bulletin 2026/34**

(21) Application number: **18735805.6**

(22) Date of filing: **04.01.2018**

(51) International Patent Classification (IPC):
***A61K 31/337*** (2006.01) ***A61K 31/7068*** (2006.01)
***A61K 9/127*** (2025.01) ***A61P 35/00*** (2006.01)
***A61K 45/06*** (2006.01) ***A61K 9/00*** (2006.01)
***A61K 9/10*** (2006.01)

(52) Cooperative Patent Classification (CPC):
(C-Sets available)
**A61P 35/00; A61K 9/0019; A61K 9/10; A61K 9/127; A61K 31/337; A61K 31/7068; A61K 45/06** (Cont.)

(86) International application number:
**PCT/CN2018/071312**

(87) International publication number:
**WO 2018/127082 (12.07.2018 Gazette 2018/28)**

(54) **TREATMENT OF REFRACTORY OR MULTIDRUG RESISTANT PANCREATIC CANCER**

BEHANDLUNG VON REFRAKTÄRER ODER MULTIRESISTENTER BAUCHSPEICHELDRÜSENKREBS

TRAITEMENT DU CANCER DU PANCRÉAS RÉFRACTAIRE OU MULTIRÉSISTANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2017 US 201762442636 P**

(43) Date of publication of application:
**13.11.2019 Bulletin 2019/46**

(73) Proprietor: **Syncore Biotechnology Co., Ltd.**
**Tung Shan Shine I-Lan 269 (TW)**

(72) Inventors:
- **HSIEH, Ruey-Kuen**
  **Tung Shan Shine I-Lan 269 (TW)**
- **TSENG, Hui-Yuan**
  **Tung Shan Shine I-Lan 269 (TW)**
- **LIN, Shi-Ting**
  **Tung Shan Shine I-Lan 269 (TW)**

(74) Representative: **Weickmann & Weickmann PartmbB**
**Postfach 860 820**
**81635 München (DE)**

(56) References cited:
**WO-A1-2005/039533 WO-A2-2006/117220**

- **FENG YANG ET AL: "Liposome based delivery systems in pancreatic cancer treatment: From bench to bedside", CANCER TREATMENT REVIEWS, ELSEVIER, AMSTERDAM, NL, vol. 37, no. 8, 21 January 2011 (2011-01-21), pages 633 - 642, XP028317499, ISSN: 0305-7372, [retrieved on 20110126], DOI: 10.1016/J.CTRV.2011.01.006**
- **KO A H ET AL: "A multinational phase 2 study of nanoliposomal irinotecan sucrosofate (PEP02, MM-398) for patients with gemcitabine-refractory metastatic pancreatic cancer", BRITISH JOURNAL OF CANCER, vol. 109, no. 4, 20 August 2013 (2013-08-20), pages 920 - 925, XP002763721, ISSN: 1532-1827**
- **EICHHORN, MARTIN E. ET AL.: "Vascular targeting by Endo TAG(TM)-1 enhances therapeutic efficacy of conventional chemotherapy in lung and pancreatic cancer", INTERNATIONAL JOURNAL OF CANCER, vol. 126, no. 5, March 2010 (2010-03-01), pages 1235 - 1245, XP055398949, ISSN: 0020-7136**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **XU, JIAMING ET AL.: "Advances in clinical research of new formulation of paclitaxel", PRACTICAL PHARMACY AND CLINICAL REMEDIES, vol. 19, no. 4, 30 April 2016 (2016-04-30), pages 510 - 517, XP009515488, ISSN: 1673-0070, DOI: 10.14053/ j.cnki.ppcr.201604033**
- **LOHR, J. M. ET AL.: "Cationic liposomal paclitaxel plus gemcitabine or gemcitabine alone in patients with advanced pancreatic cancer: a randomized controlled phase II trial.", ANNALS OF ONCOLOGY, vol. 23, no. 5, 31 May 2012 (2012-05-31), pages 1214 - 1222, XP055398947, ISSN: 0923-7534**


(52) Cooperative Patent Classification (CPC): (Cont.)

C-Sets
**A61K 31/337, A61K 2300/00;**
**A61K 31/7068, A61K 2300/00**

## Description

### TECHNICAL FIELD

The present disclosure provides a cationic liposomal formulation comprising one or more cationic lipids and a therapeutically effective amount of paclitaxel for use in a method for treating pancreatic cancer after disease progression following a treatment with one or more antineoplastic agents or other agents. The cationic liposomal formulation comprising one or more cationic lipids and a therapeutically effective amount of paclitaxel is further for use in a method for *in vivo* inhibiting the growth of multidrug resistant (MDR) pancreatic cancer cells.

### BACKGROUND

Pancreatic cancer is a highly aggressive and fatal disease with mortality rate nearly equal to incidence. As about 80% of patients are initially diagnosed with advanced disease, prognosis of pancreatic cancer is extremely poor. According to American Cancer Society, estimates of 2015 rank pancreatic cancer as the fourth leading cause of cancer-related mortality in the United States; however, it is projected to become the second leading cause of cancer death by 2030. See Cancer Res. 74: 2913-21 (2014).

For years, fluorouracil was a standard treatment for pancreatic cancer until gemcitabine showed significant improvement in median overall survival as compared with fluorouracil (5.6 v 4.4 months, P = 0.002). See Burris et al., J Clin Oncol. 15(6):2403-13 (1997). Gemcitabine has been the global standard of care for the first-line treatment of advanced pancreatic cancer since 1997. Gemcitabine is administered by intravenous infusion at a dose of 1000 mg/m$^2$ over 30 minutes once weekly for up to 7 weeks, followed by a week of rest from treatment. Subsequent cycles should consist of infusions once weekly for 3 consecutive weeks out of every 4 weeks.

Numerous studies have evaluated various regimens and combinations of gemcitabine with cytotoxic or novel targeted agents over the past decade. However, few gemcitabine-based regimens demonstrated significant improvement in overall survival. In 2007, the European Commission approved erlotinib plus gemcitabine as first-line treatment for metastatic pancreatic cancer in the EU. The US FDA previously approved this combination as a first-line treatment for patients with locally-advanced, unresectable or metastatic pancreatic cancer in 2005. A phase III study showed that the treatment with erlotinib plus gemcitabine resulted in an improvement in one-year survival compared with gemcitabine alone (23% v 17%). See J Clin Oncol. 25(15):1960-66 (2007). A combination therapy of erlotinib and gemcitabine for the treatment of pancreatic cancer includes administering 100 mg erlotinib once daily in combination with 1000 mg/m$^2$ gemcitabine once weekly.

In 2006, TS-1, a drug combination of tegafur, gimeracil and oteracil, became available in Japan for the treatment of unresectable pancreatic cancer. In a phase III study, median overall survival was 8.8 months in the gemcitabine group, 9.7 months in the TS-1 group, and 10.1 months in the gemcitabine plus S-1 group; the noninferiority of TS-1 to gemcitabine was demonstrated, whereas the superiority of gemcitabine plus TS-1 was not. See J Clin Oncol. 31(13):1640-8 (2013).

In 2010, FOLFIRINOX, a drug combination consisting of leucovorin, fluorouracil, irinotecan, and oxaliplatin, emerged as a new standard therapy. FOLFIRINOX compared with gemcitabine as first-line therapy of metastatic pancreatic cancer was studied, and the median overall survival was 11.1 months in the FOLFIRINOX group as compared with 6.8 months in the gemcitabine group. FOLFIRINOX regimen for treating pancreatic cancer, for example, is consisted of a 2-hour intravenous infusion of oxaliplatin (85 mg/m$^2$) followed by a 2-hour intravenous infusion of leucovorin (400 mg/m$^2$) concomitantly with a 90-min intravenous infusion of irinotecan (180 mg/m$^2$), followed by a bolus (400 mg/m$^2$) and a 46-hour continuous infusion (2400 mg/m$^2$) of fluorouracil. See N Engl J Med 364: 1817-25 (2011).

In 2013, albumin-bound paclitaxel was approved in the US for use in combination with gemcitabine as first-line therapy in patients with metastatic pancreatic cancer. The median overall survival was 8.5 months in the albumin-bound paclitaxel -gemcitabine group as compared with 6.7 months in the gemcitabine group. A combination therapy of albumin-bound paclitaxel and gemcitabine for the treatment of pancreatic cancer includes administering 125 mg/m$^2$ albumin-bound paclitaxel intravenously over 30-40 minutes on Days 1, 8 and 15 of each 28-day cycle, and administering 1000 mg/m$^2$ gemcitabine on Days 1, 8 and 15 of each 28-day cycle immediately after albumin-bound paclitaxel. See N Engl J Med 369:1691-703 (2013).

However, treatments for advanced pancreatic cancer over the past decade have primarily relied on only one line of therapy. This may be due to the aggressive nature of the disease and the lack of consensus on effective treatment options in second-line therapy. Therefore, there is a need in treating patients with pancreatic cancer who fail first-line treatment, such as gemcitabine alone, gemcitabine-based regimens or FOLFIRINOX. In particular, as FOLFIRINOX becomes more widely used as a first-line therapy, a second-line regimen showing efficacy and tolerability in patients who have received first-line FOLFIRINOX is required. In addition, it was reported that a low-dose continuous strategy coupled with pulsed dose may lead to the maximal delay until clinically observable resistance. See PLoS One. 2015 Nov 4;10(11). To choose proper cancer treatment, physicians need to consider multiple factors such as patient's age, medical history and

side effect. A physician's decision may include a cancer regimen which may be more liable to drug resistance, for example, a regimen lacking a pulsed dose. For those regimens, there is particularly a need of a second-line regimen. Ko et al., British Journal of Cancer, 109:920-925 (2013) discloses a multinational phase II study evaluating nanoliposomal irinotecan (PEP02 / M M-398) as second-line monotherapy in patients with gemcitabine-refractory metastatic pancreatic cancer. The study reports that liposomal irinotecan shows moderate antitumour activity with a manageable toxicity profile, achieving a median overall survival of 5.2 months and progression-free survival of 2.4 months.

Other delivery systems have been reported, such as for instance WO 2005/039533 A1, WO 2006/117220 A2, Eichhorn et al., Int. J. of Cancer, 126(5): 1235-1245 (2010), Xu et al., Practical Pharmacy and Clinical Remedies, 19(4):510-517 (2016), Löhr et al., Annals of Oncology 23(5):1214-1222 (2012), and Feng Yang et al., Cancer Treatment reviews 37(8):633-642 (2011), which describe cationic liposomal preparation comprising paclitaxel in combination with gemcitabine for use in the treatment of pancreatic cancers, in particular inoperable metastatic pancreatic tumors, and more particularly in the treatment of advanced and /or metastatic adenocarcinoma of the pancreas.

## SUMMARY

Provided herein is a cationic liposomal formulation comprising one or more cationic lipids and a therapeutically effective amount of paclitaxel for use in a method for treating refractory or resistant pancreatic cancer comprising administering to a subject in need thereof a cationic liposomal formulation comprising one or more cationic lipids and a therapeutically effective amount of paclitaxel. In embodiments, the cationic liposomal formulation is administered in combination with a therapeutically effective amount gemcitabine. The cationic liposomal formulation and gemcitabine are a combination therapy and are administered simultaneously but separately or sequentially. In particular embodiments, the pancreatic cancer is refractory or resistant to a certain therapy, such as a first-line or second-line therapy. The cationic liposomal formulation provided herein can be used as second-line or third-line therapy.

In embodiments of the invention described herein, the subject in need thereof has been treated with a certain therapy and the pancreatic cancer has become refractory or resistance to the therapy. The subject has not been treated with any other therapies other than the therapy that the pancreatic cancer has become refractory or resistant.

In some embodiments, the subject has previously been treated with one or more antineoplastic agents comprising fluorouracil, bleomycin, bortezomib, carboplatin, cisplatin, cytarabine, docetaxel, doxorubicin, elmustin, erlotinib, etoposide, gemcitabine, idarubicin, imatinib, lomustine, methotrexate, mitomycin, mitoxantrone, oxaliplatin, paclitaxel, pemetrexed, sunitinib, topotecan, treosulfan, vemurafenib, vinblastine, vincristine, vindesine, or vinorelbine.

In some embodiments, the subject has previously been treated with fluorouracil-based combination therapy, particularly a combination of oxaliplatin, leucovorin, irinotecan, and fluorouracil.

In some embodiments, the subject has previously been treated with gemcitabine-based combination therapy.

In some embodiments, the subject has previously been treated with an antimitotic agent selected from a group consisting of paclitaxel, docetaxel, vinblastine, vincristine, vindesine and vinorelbine. In some embodiments, the subject has previously been treated with a growth factor inhibitor selected from a group consisting of erlotinib, cetuximab, gefinitib, imatinib, panitumumab, sunitinib and vemurafenib.

In a preferred embodiment, the cationic liposomal formulation comprising paclitaxel for use in a method for treating refractory or resistant pancreatic cancer is administered on days 1, 4, 8, 11, 15, 18, 22, 25, 29, 32, 36, 39, 43 and 46 at a dose of 1 to 60 mg/m$^2$ and gemcitabine at a dose of 300 to 1500 mg/m$^2$ is administered on days 4, 11, 18, 25, 32, 39 and 46 of a treatment cycle of seven weeks.

In a preferred embodiment, the cationic liposomal formulation for use in a method for treating refractory or resistant pancreatic cancer is provided, wherein a first treatment cycle is followed by one or more subsequent treatment cycles. The first treatment cycle is a period of seven weeks, while each subsequent treatment cycle is a period of three weeks. A dosing interval between the first treatment cycle and a subsequent cycle and between two subsequent treatment cycles is one week.

In a preferred embodiment, in the first treatment cycle, the cationic liposomal formulation comprising paclitaxel at a dose of 1 to 60 mg/m$^2$ is administered on days 1, 4, 8, 11, 15, 18, 22, 25, 29, 32, 36, 39, 43 and 46 and gemcitabine at a dose of 300 to 1500 mg/m$^2$ is administered on days 4, 11, 18, 25, 32, 39 and 46. A dosing interval between the first treatment cycle and the subsequent treatment cycle is one week. In the subsequent treatment cycles, the cationic liposomal formulation comprising paclitaxel at a dose of 1 to 60 mg/m$^2$ is administered on days 1, 4, 8, 11, 15 and 18 and gemcitabine at a dose of 300 to 1500 mg/m$^2$ is administered on days 4, 11 and 18. A dosing interval between two subsequent treatment cycles is one week.

In a preferred embodiment, 1 mg/m$^2$ to 60 mg/m$^2$ paclitaxel comprised in the cationic liposomal formulation for use in a method for treating refractory or resistant pancreatic cancer and 300 mg/m$^2$ to 1500 mg/m$^2$ gemcitabine are administered to the subject.

In a preferred embodiment, 11 mg/m$^2$ to 22 mg/m$^2$ paclitaxel comprised in the cationic liposomal formulation for use in a method for treating refractory or resistant pancreatic cancer and 500 mg/m$^2$ to 1000 mg/m$^2$ gemcitabine are

administered to the subject.

Also provided herein is a cationic liposomal formulation comprising one or more cationic lipids and a therapeutically effective amount of paclitaxel for use in a method for *in vivo* inhibiting the growth of MDR pancreatic cancer cells comprising administering to MDR pancreatic cells a cationic liposomal formulation comprising one or more cationic lipids and a therapeutically effective amount of paclitaxel.

In a preferred embodiment, the cationic liposomal formulation for use in a method for *in vivo* inhibiting the growth of MDR pancreatic cancer cells, wherein the method further comprises administering a therapeutically effective amount of gemcitabine, more preferably wherein the cationic liposomal formulation and the therapeutically effective amount of gemcitabine are administered simultaneously, but separately, or are administered sequentially.

## DETAILED DESCRIPTION

As used herein, the term "therapeutically effective amount" is an amount of an active agent that is sufficient to achieve the desired therapeutic result in the treated subject. The result can be reduction, amelioration, palliation, lessening, delaying, and/or alleviation of one or more of the signs, symptoms, or causes of a disease. In some aspects, a therapeutically effective amount comprises an amount sufficient to cause a tumor to shrink or to decrease growth rate. In some aspect, a therapeutically effective amount is an amount sufficient to prevent or delay tumor recurrence. In some aspects, a therapeutically effective amount is an amount sufficient to inhibit, retard, slow to some extent and may stop cancer cell infiltration into peripheral organs; (iv) inhibit (i.e., slow to some extent and may stop) tumor metastasis; (v) inhibit tumor growth; (vi) prevent or delay occurrence and/or recurrence of tumor. A therapeutically effective amount can be administered in one or more administrations.

As used herein, the term "subject" is a human cancer patient. Subjects in need of a treatment (in need thereof) are subjects having pancreatic cancer. In embodiments, the subject is a human patient diagnosed with or suffering from pancreatic cancer. In particular embodiments, the subject is refractory or resistant to first-line or second-line therapy for pancreatic cancer and is in need of a second-line or third-line therapy for treatment of pancreatic cancer.

The term "pancreatic cancer" as used herein includes "locally advanced pancreatic cancer" and "metastatic pancreatic cancer." "Locally advanced pancreatic cancer" refers to tumors that arise in pancreatic exocrine or neuroendocrine tissue, but distant metastases are absent. In contrast, "metastatic pancreatic cancer" refers to cancer spreading from the site from which it originates in the pancreas to involve another part of the body, for example, liver. Cancers originating from pancreatic exocrine tissue include acinar cell carcinomas, adenocarcinomas, adenosquamous carcinomas, ampullary cancers, colloid carcinomas, giant cell tumors, hepatoid carcinomas, intraductal papillary-nmeinous neoplasms, mucinous cystadenocarcinomas, pancreatoblastomas, serous cystadenocarcinomas, signet ring cell carcinomas, solid and pseudopapillary tumors, and undifferentiated carcinomas. Cancers originating from neuroendocrine tissue include gastrinomas, glucagonomas, insulinomas, nonfunctional islet cell tumors, somatostatinomas and vasoactive intestinal peptide-releasing tumors. Locally advanced pancreatic cancer is an adenocarcinoma. In further aspect, the adenocarcinoma is ductal adenocarcinoma.

The term "growth factor inhibitor" includes erlotinib, cetuximab, gefinitib, imatinib, panitumumab, sunitinib and vemurafenib.

The term "antimitotic agent" includes paclitaxel, docetaxel, vinblastine, vincristine, vindesine and vinorelbine.

The term "first-line therapy" refers to the standard treatment given to a subject diagnosed with a disease. It is the initial treatment and usually accepted as the best treatment for the diagnosis.

The term "second-line therapy" refers to a treatment which is chosen after first-line treatment has failed to achieve its goal, or has side effects requiring a subject to stop using that treatment. The second-line therapy is usually used when the first-line treatment has failed, was effective previously but has since stopped working, or has side effects that are not tolerated by the subject. The term "third-line therapy" refers to treatment that is given when both the first-line therapy and the second-line therapy has failed.

As used herein a "dosage regimen" refers to a protocol used to administer a liposomal formulation or non-liposomal formulation to a subject. A dosage regimen comprises a dose and dosing interval. A dosage regimen further comprises a dosing duration. As used herein "dose" refers to an amount of an active agent given in a single administration. The interval between doses can be a desired amount of time and is referred to as the "dosing interval". As used herein "dosing duration" refers to the period of time over which a dose is administered. As used herein, "pulsed dose" refers to a dose sharply releasing an active agent once or repeatedly. In some aspects, a pulsed dose is a bolus dose.
The unit "mg/m$^2$" refers to an amount of an active agent per human body surface area (m$^2$). The dose calculation refers only to the mass of the active agent (not to the lipid portion).

The term "combination therapy" as used herein includes simultaneous administration of at least two active agents to a subject or their sequential administration within a time period during which the first administered therapeutic agent is still present in the subject when the second administered therapeutic agent is administered. Combination therapy as used herein also includes administering the at least two active agents separately but at the same time.

The term "fluorouracil-based combination therapy" includes a combination of oxaliplatin, leucovorin, irinotecan, and fluorouracil, and a combination of leucovorin, liposomal irinotecan, and fluorouracil.

The term "gemcitabine-based combination therapy" includes a combination of albumin-bound paclitaxel and gemcitabine, a combination of erlotinib and gemcitabine, a combination of capecitabine and gemcitabine, and a combination of cisplatin and gemcitabine.

The term "resistant" or "refractory" refers to cancer cells that survive after treating with an active agent. Such cells may have responded to an active agent initially, but subsequently exhibited a reduction of responsiveness during treatment, or did not exhibit an adequate response to the active agent in that the cells continued to proliferate in the course of treatment with the active agent.

The term "liposome" refers to a microscopic spherical membrane-enclosed vesicle (about 50-2000 nm diameter). The term "liposome" encompasses any compartment enclosed by a lipid bilayer. Liposomes are also referred to as lipid vesicles. In order to form a liposome, the lipid molecules comprise elongated non polar (hydrophobic) portions and polar (hydrophilic) portions. The hydrophobic and hydrophilic portions of the molecule are preferably positioned at the two ends of an elongated molecular structure. When such lipids are dispersed in water they spontaneously form bilayer membranes referred to as lamellae. The lamellae are composed of two mono layer sheets of lipid molecules with their nonpolar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell.

Thus, the bilayer of a liposome has similarities to a cell membrane without the protein components present in a cell membrane. As used herein, the term liposome includes multilamellar liposomes, which generally have a diameter in the range of about 1 to 10 micrometers and having anywhere from two to hundreds of concentric lipid bilayers alternating with layers of an aqueous phase, and also includes unilamellar vesicles which are a single lipid layer and have a diameter in the range of about 20 to about 400 nanometers (nm), about 50 to about 300 nm, about 300 to about 400 nm, or about 100 to about 200 nm, which vesicles can be produced by subjecting multilamellar liposomes to ultrasound, by extrusion under pressure through membranes having pores of defined size, or by high pressure homogenization. The liposomes can be unilamellar vesicles, which have a single lipid bilayer, and a diameter in the range of about 25-400 nm.

The cationic liposomal formulation provided herein includes one or more cationic lipids, paclitaxel, and optionally a neutral and/or anionic lipid. As used herein, the terms "liposome", "liposomal preparation", and "liposomal formulation" are used synonymously throughout the present application.

In a preferred embodiment, the amount of cationic lipids in the cationic liposomal formulation is from 30 mole% to 99.9 mole%. The amount of paclitaxel in the cationic liposomal formulation is at least 0.1 mole%. The amount of neutral and/or anionic lipid is from 30 mole % to 55 mole%.

The amount of cationic lipids in the cationic liposomal formulation includes from about 40 mole% to about 95 mole%, about 50 mole% to about 90 mole%, about 60 mole% to about 85 mole%, about 65 mole% to about 75 mole%, or about 70 mole%.

The cationic liposomal formulation includes paclitaxel in an amount of from about 0.5 mole% to about 10 mole%, about 1.0 mole% to about 8 mole%, about 2 mole% to about 6 mole%, about 5 mole%, about 2.5 mole%, or about 3.0 mole%.

Optionally, the cationic liposomal formulation includes neutral and/or anionic lipids, in an amount of from about 30 mole% to about 70 mole%, about 40 mole% to about 60 mole%, about 45 mole%, or about 55 mole%.

The cationic liposomal formulation has a zeta potential in the range of about 0 mV to about 100 mV or in the range of about 20 mV to about 100 mV. In a preferred embodiment, the cationic liposomal formulation for use in a method for treating refractory or resistant pancreatic cancer has a positive zeta potential in 0.05 mM KCl solution at pH 7.5 at room temperature.

As used herein, the term "zeta potential" refers to a measured electrical potential of a particle, such as a liposome, measured with an instrument, such as a Zetasizer 3000 using Laser Doppler micro-electrophoresis under the conditions specified. The zeta potential describes the potential at the boundary between bulk solution and the region of hydrodynamic shear or diffuse layer. The term is synonymous with "electrokinetic potential" because it is the potential of the particles which acts outwardly and is responsible for the particle's electrokinetic behavior.

The one or more cationic lipids in the cationic liposomal formulation are selected from the group consisting of N-[1-(2,3-dioleoyloxy)propyl]-N,N,N-trimethyl ammonium salts, such as N-[1-(2,3-dioleoyloxy)propyl]-N,N,N-trimethyl ammonium salt (DOTAP); dimethyldioctadecyl ammonium bromide (DDAB); 1,2-diacyloxy-3-trimethylammonium propanes, including for example, dioleoyl, dimyristoyl, dilauroyl, dipalmitoyl, and distearoyl, and including those with two different acyl chain linked to the glycerol backbone); N-[1-(2,3-dioloyloxy)propyl]-N,N-dimethyl amine (DODAP); 1,2-diacyloxy-3-dimethylammonium propanes, including for example, dioleoyl, dimyristoyl, dilauroyl, dipalmitoyl, and distearoyl, and including those with two different acyl chain linked to the glycerol backbone; N-[1-(2,3-dioleyloxy)propyl]-N,N,N-trimethylammonium chloride (DOTMA); 1,2-dialkyloxy-3-dimethylammonium propanes, including for example dioleyl, dimyristyl, dilauryl, dipalmityl, and distearyl and including those with two different alkyl chain linked to the

glycerol backbone; dioctadecylamidoglycylspermine (DOGS); 3β-[N--(N',N'-dimethylamino-ethane)carbamoyl]cholesterol (DC-Chol); 2,3-dioleoyloxy-N-(2-(sperminecarboxamido)-ethyl)-N,N-dimethyl-1-propanam-inium trifluoro-acetate (DOSPA); β-alanyl cholesterol; cetyl trimethyl ammonium bromide (CTAB); diC14-amidine; N-tert-butyl-N'-tetradecyl-3-tetradecylamino-propionamidine; 14Dea2; N-(alpha-trimethylammonioacetyl)didodecyl-D-glutamate chloride (TMAG); O,O'-ditetradecanoyl-N-(trimethylammonio-acetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butan-ediammonium iodide; 1-[2-(acyloxy)ethyl]-alkyl(alkenyl)-3-(2-hydroxyethyl)-imidazolinium chloride derivatives, such as 1-[2-(9(Z)-octadecenoyloxy)ethyl]-2-(8(Z)-heptadecenyl-3-(2-hydroxyethyl)-imidazolinium chloride (DOTIM) and 1-[2-(hexadecanoyloxy) ethyl]-2-pentadecyl-3-(2-hydroxyethyl)imidazolinium chloride (DPTIM); 2,3-dialkyloxypropyl quaternary ammonium compound derivatives, containing a hydroxyalkyl moiety on the quaternary amine, for example, 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimethyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), and 1,2-disteryloxypropyl-3-dimethyl-hydroxyethyl ammonium bromide (DSRIE); cationic esters of acyl carnitines; and cationic triesters of phospahtidylcholine, for example, 1,2-diacyl-sn-glycerol-3-ethylphosphocholines, in which the hydrocarbon chains are saturated or unsaturated and branched or unbranched with a chain length from $C_{12}$ to $C_{24}$, and the two acyl chains may or may not be identical.

In a preferred embodiment, the cationic lipid is N-[1-(2,3-dioleoyloxy)propyl]-N,N,N-trimethyl ammonium salt (DOTAP); dimethyldioctadecyl ammonium bromide (DDAB); 1,2-diacyloxy-3-trimethylammonium propane N-[1-(2,3-dioloyloxy)propyl]-N, N-dimethyl amine (DODAP); 1,2-diacyloxy-3-dimethylammonium propane; N-[1-(2,3- dioleyloxy) propyl]-N,N,N-trimethylammonium chloride (DOTMA); 1,2-dialkyloxy-3- dimethylammonium propane; dioctadecylamidoglycylspermine (DOGS); 3β-[N- (N',N'-dimethylamino-ethane)carbamoyl]cholesterol (DC-Chol); 2, 3-dioleoyloxy-N-(2-(sperminecarboxamido)-ethyl)-N, N-dimethyl-1-propanaminium trifluoroacetate (DOSPA); β-alanyl cholesterol; cetyl trimethyl ammonium bromide (CTAB); diC14- amidine; N-tert-butyl-N'-tetradecyl-3-tetradecylamino-propionamidine; 14Dea2; N- (alpha-trimethylammonioacetyl)didodecyl-D-glutamate chloride (TMAG); O,O'- ditetradecanoyl-N-(trimethylammonioacetyl)diethanolamine chloride; 1,3-dioleoyloxy-2-(6-carboxy-spermyl)-propylamide (DOSPER); N,N,N',N'-tetramethylN,N'-bis(2-hydroxylethyl)-2,3-dioleoyloxy-1,4-butanediammonium iodide; 1-[2- (acyloxy)ethyl]2-alkyl (alkenyl)-3-(2-hydroxyethyl)-imidazolinium chloride; 1,2- dioleoyl-3-dimethyl-hydroxyethylammonium bromide (DORI); 1,2-dioleyloxypropyl3-dimethylhydroxyethylammonium bromide (DORIE); 1,2-dioleyloxypropyl-3- dimethylhydroxypropylammonium bromide (DORIE-HP); 1,2-dioleyloxypropyl-3-dimethylhydroxybutylammonium bromide (DORIE-HS); 1,2-dioleyloxypropyl-3-dimethylhydroxypentylammonium bromide (DORIE-Hpe); 1,2-dimyristyloxypropyl3-dimethylhydroxylethylammonium bromide (DMRIE); 1,2-dipalmityloxypropyl-3-dimethylhydroxyethylammonium bromide (DPRIE); 1,2-disteryloxypropyl-3-dimethylhydroxyethylammonium bromide (DSRIE); or 1,2-diacyl-sn-glycerol-3- ethylphosphocholine, and more preferably the 1-[2-(acyloxy)ethyl]2-alkyl (alkenyl)-3-(2-hydroxyethyl)- imidazolinium chloride is 1-[2-(9(Z)-octadecenoyloxy)ethyl]-2-(8(Z)-heptadecenyl3-(2-hydroxyethyl)-imidazoliniumchloride (DOTIM) or 1-[2-(hexadecanoyloxy)ethyl]-2-pentadecyl-3-(2-hydroxyethyl)imidazolinium chloride (DPTIM).Optionally, the liposomal preparation comprises one or more neutral and/or anionic lipids. The neutral and anionic lipids are selected from sterols or lipids such as cholesterol, phospholipids, lysolipids, lysophospholipids, sphingolipids, or pegylated lipids with a neutral or negative net change. In particular aspects, the neutral and anionic lipids include: phosphatidylserine; phosphatidylglycerol; phosphatidylinositol; fatty acids; sterols containing a carboxylic acid group for example, cholesterol; 1,2-diacyl-sn-glycero-3-phosphoethanolamines, including DOPE; 1,2-diacyl-glycero-3-phosphocholines; and sphingomyelin. The fatty acids linked to the glycerol backbone have various length and number of double bonds. Phospholipids can have two different fatty acids. The neutral and/or anionic lipids are in the liquid crystalline state at room temperature and they are miscible with the used cationic lipid, in the ratio as they are applied. The neutral and/or anionic lipids and the cationic lipids can form a uniform phase and no phase separation or domain formation occurs. In a preferred embodiment, the neutral lipid is (a) cholesterol, phospholipid, lysolipid, sphingolipid, or pegylated lipid with a neutral charge, (b) lysophospholipid, or (c) 1,2-diacyl-sn-glycero-3-phosphoethanolamine, 1,2-diacyl-sn-glycero-3- phosphocholine, or sphingomyelin, particularly wherein 1,2-diacyl-sn-glycero-3-phosphoethanolamine is DOPE (1,2-dioleoyl-sn-glycero-3-phosphoethanolamine) or DOPC (1,2-dioleoyl-sn-glycero-3-phosphocholine).

The cationic liposomal formulation includes taxanes. As used herein, the term "taxane" herein refers to a class of antineoplastic agents having the function of binding microtubules which inhibit cell division and having a structure that includes the taxane ring structure and a stereospecific side chain that is required for cytostatic activity. The term taxane also includes a variety of known derivatives, such as hydrophilic derivatives and hydrophobic derivatives. Taxane derivatives include galactose and mannose derivatives described in International Patent Application No. WO 99/18113; piperazino and other derivatives described in WO 99/14209; taxane derivatives described in WO 99/09021, WO 98/22451, and U.S. Pat. No. 5,869,680; 6-thio derivatives described in WO 98/28288; sulfenamide derivatives described in U.S. Pat. No. 5,821,263; and taxol derivative described in U.S. Pat. No. 5,415,869. Examples of

taxanes include paclitaxel, docetaxel, and carbazitaxel.

The term "paclitaxel" includes analogues, formulations, and derivatives such as, for example, docetaxel (Taxotere, a formulation of docetaxel), 10-desacetyl analogs of paclitaxel and 3'N-desbenzoyl-3'N-t-butoxycarbonyl analogs of paclitaxel. Paclitaxels can be readily prepared utilizing techniques known to those skilled in the art (see also WO 94/07882, WO 94/07881, WO 94/07880, WO 94/07876, WO 93/23555, WO 93/10076; U.S. Pat. Nos. 5,294,637; 5,283,253; 5,279,949; 5,274,137; 5,202,448; 5,200,534; 5,229,529; and EP 590,267), or obtained from a variety of commercial sources, including for example, Sigma Chemical Co., St. Louis, Mo. (T7402 from Taxus brevifolia; or T-1912 from Taxus yannanensis). Paclitaxel refers not only to the common chemically available form of paclitaxel (e.g. Taxol®), but also analogs (e.g., Taxotere, as noted above) and paclitaxel conjugates (e.g., paclitaxel-PEG, paclitaxel-dextran, or paclitaxel-xylose).

The term "derivative" refers to a compound derived from some other compound while maintaining its general structural features. Derivatives may be obtained for example by chemical functionalization or derivatization.

The term "liposomal paclitaxel" or "lipid complexed paclitaxel" refers to a liposomal preparation. A specific liposomal paclitaxel formulation is EndoTAG®-1. The manufacture of such a formulation is disclosed in WO 2004/002468. EndoTAG®-1 is a liposomal preparation with a mole ratio of 50:47:3 mole % of DOTAP, DOPC and paclitaxel.

Due to the steric shape and the amphiphilic nature of the lipids, self-assembly leads to the formation of lipid bilayers (membranes), in which the hydrophobic alkyl chains are oriented toward each other and the polar head groups are oriented toward the aqueous phase. These membranes are organized as spherical vesicles, so-called liposomes. Because of the presence of cationic (positively charged) lipid molecules in the bilayer membrane, the liposomes are cationic. EndoTAG®-1 is delivered as a lyophilized powder for solution for infusion. It is reconstituted with water for injection prior to application. The resulting solution consists of small liposomal vesicles with an intensity weighted average particle size < 300 nm.

The cationic liposomal formulation described herein includes one or more cationic lipids, one or more neutral lipids, and paclitaxel. In a preferred embodiment, the cationic lipid is DOTAP; the neutral lipid is DOPC. In a preferred embodiment, the cationic liposomal formulation for use in a method for treating refractory or resistant pancreatic cancer comprises DOTAP, DOPC, and paclitaxel. The mole ratio of cationic lipids, neutral lipids, and taxanes is in the range of about 40 to 60 cationic lipids, about 39 to 55 neutral lipids, and about 1 to 5 paclitaxel. In a particular embodiment, the cationic liposomal formulation includes DOTAP, DOPC, and paclitaxel in a mole ratio of 50:47:3.

The cationic liposomal formulation can include one or more carriers. As used herein, the term "carrier" refers to a diluent, adjuvant, excipient, or vehicle which is suitable for administering a diagnostic or therapeutic agent. The term also refers to a pharmaceutically acceptable carrier that contain, complexes or is otherwise associated with an agent to facilitate the transport of such an agent to its intended target site. Carriers include those known in the art, such as liposomes, polymers, lipid complexes, serum albumin, antibodies, cyclodextrins, dextrans, chelates, or other supramolecular assemblies.

The formulations, in particular the cationic liposomal formulation, disclosed herein can be in a dry, dehydrated, or lyophilized form. Prior to administration, the formulation can be hydrated in pharmaceutical grade water or saline or another suitable liquid, preferably comprising physiologically acceptable carriers such as a buffer.

The formulations disclosed herein can be in the form of kits. The kit can include a cationic liposomal formulation and one or more active agents described herein. The one or more active agents can be a chemotherapeutic agent. The non-liposomal formulation in the kit includes a taxane, such as paclitaxel, and the active agent is gemcitabine. The kits described herein can also include a container and/or reagents for preparing the formulations for administration. As an example, the cationic liposomal formulation can be in a dehydrated form that can be reconstituted by hydration.

As used herein, the term "combination" or "co-administration" refers to an administration schedule that is synchronous, serial, overlapping, alternating, parallel, or any other treatment schedule in which the various active agents or therapies are administered as part of a single treatment regimen, prescription or indication or in which the time periods during which the various agents or therapies that are administered otherwise partially or completely coincide.

Depending on the duration of the treatment and on the observed side effects, the administration of the formulations can also be omitted for at least one week or several weeks during the treatment period.

The methods described herein include administering the cationic liposomal formulation in a single dose of from about 1 mg/m$^2$ to about 60 mg/m$^2$.

As used herein, the unit mg/m$^2$, refers to mg of active agent, for example paclitaxel, per m$^2$ body surface area (bsa) of the subject.

As used herein, the unit mg/kg body weight of a subject or mg/kg refers to mg of active agent, for example paclitaxel, per kg body weight (bw) of a subject.

On an average, a human subject has a body surface area of about 1.84 m$^2$. Thus, for an average person of 70 kg body weight and 172 cm height, values for single doses, monthly doses, etc. which are in mg/kg body weight (bw) may be converted for human applications to corresponding values of in mg/m$^2$ human body surface area (bs) by multiplication with a species-specific factor according to known methods. Similarly, doses in mg/m$^2$ bs of a human subject can be converted to

mg/kg bw of a human subject.

Provided herein is a cationic liposomal formulation for use in a method for treating a subject having pancreatic cancer and the subject is refractory or resistant to a first-line or second-line therapy. In embodiments, the first-line or second-line therapy includes administering an antineoplastic agent, a combination therapy, gemcitabine-based therapy, a fluorouracil-based combination therapy, a growth factor inhibitor therapy, or an antimitotic agent therapy.

In embodiments, the first-line or second-line therapy includes administering one or more antineoplastic agents comprising fluorouracil, bleomycin, bortezomib, carboplatin, cisplatin, cytarabine, docetaxel, doxorubicin, elmustin, erlotinib, etoposide, gemcitabine, idarubicin, imatinib, lomustine, methotrexate, mitomycin, mitoxantrone, oxaliplatin, paclitaxel, pemetrexed, sunitinib, topotecan, treosulfan, vemurafenib, vinblastine, vincristine, vindesine, or vinorelbine.

In some embodiments, the subject is refractory or resistant to a gemcitabine-based therapy, such as gemcitabine-based combination therapy.

In some aspects, the subject is refractory or resistant to a combination of albumin-bound paclitaxel and gemcitabine a combination of erlotinib and gemcitabine, a combination of capecitabine and gemcitabine, or a combination of cisplatin and gemcitabine.

In some embodiments, the subject is refractory or resistant to a fluorouracil-based combination therapy.

In some embodiments, the subject is refractory or resistant to a combination of oxaliplatin, leucovorin, irinotecan and fluorouracil.

In some embodiments, the subject is refractory or resistant to a growth factor inhibitor such as erlotinib, cetuximab, gefinitib, imatinib, panitumumab, sunitinib, or vemurafenib.

In some embodiments, the subject is refractory or resistant to an antimitotic agent such as paclitaxel, docetaxel, vinblastine, vincristine, vindesine, or vinorelbine.

In a preferred embodiment, the subject is in need of a second-line treatment and has previously been treated with an intravenous infusion of 70 mg/m$^2$ to 100 mg/m$^2$ oxaliplatin followed by an intravenous infusion of 300 mg/m$^2$ to 500 mg/m$^2$ leucovorin concomitantly with an intravenous infusion of 90 mg/m$^2$ to 270 mg/m$^2$ irinotecan, followed by an intravenous bolus of 300 mg/m$^2$ to 800 mg/m$^2$ fluorouracil and an intravenous infusion of 1200 mg/m$^2$ to 3600 mg/m$^2$ fluorouracil.

In a preferred embodiment, the subject has previously been treated with an intravenous infusion of 85 mg/m$^2$ oxaliplatin followed by an intravenous infusion of 400 mg/m$^2$ leucovorin concomitantly with an intravenous infusion of 180 mg/m$^2$ irinotecan, followed by an intravenous bolus of 400 mg/m$^2$ fluorouracil and an intravenous infusion of 2400 mg/m$^2$ fluorouracil.

In a preferred embodiment, the subject is in need of a second-line or third-line treatment and has previously been treated with one or more antineoplastic agents without administering a pulsed dose.

In a preferred embodiment, the subject is in need of a second-line or third-line treatment and has previously been treated with an intravenous infusion of 70 mg/m$^2$ to 100 mg/m$^2$ oxaliplatin followed by an intravenous infusion of 300 mg/m$^2$ to 500 mg/m$^2$ leucovorin concomitantly with an intravenous infusion of 90 mg/m$^2$ to 180 mg/m$^2$ irinotecan, followed by an intravenous infusion of 1200 mg/m$^2$ to 3600 mg/m$^2$ fluorouracil.

In a preferred embodiment, the subject is in need of a second-line or third-line treatment and has previously been treated with an intravenous infusion of 85 mg/m$^2$ oxaliplatin followed by an intravenous infusion of 400 mg/m$^2$ leucovorin concomitantly with an intravenous infusion of 130 mg/m$^2$ to 150 mg/m$^2$ irinotecan, followed by an intravenous infusion of 2400 mg/m$^2$ fluorouracil.

In the methods described herein, the cationic liposomal formulation is administered in a dose of from about 1 mg/m$^2$ to about 50 mg/m$^2$, about 25 mg/m$^2$ to about 50 mg/m$^2$, about 10 mg/m$^2$ to about 25 mg/m$^2$, or from about 11 mg/m$^2$ to about 22 mg/m$^2$ of the body surface area (bsa) of the subject. The cationic liposomal formulation is administered at a dose of about 1 mg/m$^2$, about 2.5 mg/m$^2$, about 5 mg/m$^2$, about 7.5 mg/m$^2$, 11 mg/m$^2$, about 22 mg/m$^2$, about 25 mg/m$^2$, about 28 mg/m$^2$, about 31 mg/m$^2$, about 33 mg/m$^2$, about 35 mg/m$^2$, about 38 mg/m$^2$, about 41 mg/m$^2$, about 44 mg/m$^2$, or about 47 mg/m$^2$ of the bsa of the subject.

Gemcitabine is administered at a dose from about 100 mg/m$^2$ to about 1500 mg/m$^2$, about 100 mg/m$^2$ to about 500 mg/m$^2$, about 500 mg/m$^2$ to about 1500 mg/m$^2$, about 600 mg/m$^2$ to about 1400 mg/m$^2$, about 700 mg/m$^2$ to about 1300 mg/m$^2$, about 800 mg/m$^2$, or about 1250 mg/m$^2$ bsa of the subject. In particular, gemcitabine is administered at a dose of about 500 mg/m$^2$ or 1000 mg/m$^2$.

In a preferred embodiment, the cationic liposomal formulation for use in a method for treating refractory or resistant pancreatic cancer is administered twice weekly, and gemcitabine is administered once weekly.

In a preferred embodiment, the cationic liposomal formulation comprising paclitaxel for use in a method for treating refractory or resistant pancreatic cancer is administered on days 1, 4, 8, 11, 15, 18, 22, 25, 29, 32, 36, 39, 43 and 46 at a dose of 1 to 60 mg/m$^2$ and gemcitabine at a dose of 300 to 1500 mg/m$^2$ is administered on days 4, 11, 18, 25, 32, 39 and 46 of a treatment cycle of seven weeks.

In a preferred embodiment, the cationic liposomal formulation comprising paclitaxel for use in a method for treating pancreatic cancer are provided, wherein the method comprises a first treatment cycle, which is followed by one or

more subsequent treatment cycles. The first treatment cycle is a period of seven weeks, while each subsequent treatment cycle is a period of three weeks. In the first treatment cycle, the cationic liposomal formulation comprising paclitaxel at a dose of 1 to 60 mg/m$^2$ is administered on days 1, 4, 8, 11, 15, 18, 22, 25, 29, 32, 36, 39, 43 and 46 and gemcitabine at a dose of 300 to 1500 mg/m$^2$ is administered on days 4, 11, 18, 25, 32, 39 and 46. A dosing interval between the first treatment cycle and the subsequent treatment cycle is one week. In the subsequent treatment cycles, the cationic liposomal formulation comprising paclitaxel at a dose of 1 to 60 mg/m$^2$ is administered on days 1, 4, 8, 11, 15 and 18 and gemcitabine at a dose of 300 to 1500 mg/m$^2$ is administered on days 4, 11 and 18. A dosing interval between two subsequent treatment cycles is one week.

In a preferred embodiment, 1 mg/m$^2$ to 60 mg/m$^2$ paclitaxel comprised in the cationic liposomal formulation for use in a method for treating refractory or resistant pancreatic cancer and 300 mg/m$^2$ to 1500 mg/m$^2$ gemcitabine are administered to the subject.

In a more preferred embodiment, 11 mg/m$^2$ to 22 mg/m$^2$ paclitaxel in the cationic liposomal formulation for use in a method for treating refractory or resistant pancreatic cancer and 500 mg/m$^2$ to 1000 mg/m$^2$ gemcitabine are administered to the subject.

In another preferred embodiment, the cationic liposomal formulation for use in a method for treating refractory or resistant pancreatic cancer is administered to the subject at a rate of 0.5 mL/min for first 15 minutes, followed by a rate of 1.0 mL/min for second 15 minutes, and followed by a rate of 1.5 mL/min after 30 minutes.

Gemcitabine can be applied at a lower weekly dose compared to that in the standard of care for pancreatic cancer (1000 mg/m$^2$). In someaspects, gemcitabine is administered at a dose of about 500 mg/m$^2$, 550 mg/m$^2$, 600 mg/m$^2$, 650 mg/m$^2$, 700 mg/m$^2$, 750 mg/m$^2$, or 800 mg/m$^2$.

The continued administration of lower doses once or twice weekly is at least as effective as the administration of a single high dose or frequent low dose administration interrupted by pause intervals. Depending on the effectiveness of the dosage regimen, the doses of the formulations and the dosing intervals may remain constant, increased, or decreased during the treatment period.

In a preferred embodiment, a neoadjuvant therapy is administered before the cationic liposomal formulation for use in a method for treating refractory or resistant pancreatic cancer. The neoadjuvant therapy refers to a treatment given as a first step to shrink a tumor before the main treatment, for example surgery, is given. A neoadjuvant therapy includes chemotherapy, radiation therapy, and hormone therapy. As an example, the treatment of pancreatic cancer includes a neoadjuvant therapy including administering FOLFIRINOX, followed by surgery, which is followed by a method described herein.

In another preferred embodiment, a neoadjuvant therapy is administered before the cationic liposomal formulation for use in a method for *in vivo* inhibiting the growth of MDR pancreatic cells.

The methods disclosed herein are characterized by selective targeting, improved efficacy, reduced adverse side effects as compared to conventional chemotherapy, reduced disease related pain, improved quality of life, stabilization of body weight during treatment, and synergistic effects with other therapy regimens.

Described are methods for *in vivo* inhibiting the growth of pancreatic cancer cells that are refractory or resistant to one or more (a combination of) antineoplastic agents, for example multidrug resistant (MDR) cells. The antineoplastic agents are fluorouracil, bleomycin, bortezomib, carboplatin, cisplatin, cytarabine, docetaxel, doxorubicin, elmustin, erlotinib, etoposide, gemcitabine, idarubicin, imatinib, lomustine, methotrexate, mitomycin, mitoxantrone, oxaliplatin, paclitaxel, pemetrexed, sunitinib, topotecan, treosulfan, vemurafenib, vinblastine, vincristine, vindesine and vinorelbine. Examples of MDR pancreatic cancer cells include PAXF 546, PAXF 1986, PACF 1998, PACF 2005, PAXF 2035, PAXF 2059, PAXF CAPAN-2, and PACF PANC-1.

The MDR pancreatic cancer cells are in vitro cells, in vivo cells, ex vivo cells, or cells obtained from a xenograft.

## EXAMPLE

Example 1: Multidrug Resistant Cells

Pancreatic cancer cell lines resistant to one or more agents such as fluorouracil, bleomycin, bortezomib, carboplatin, cisplatin, cytarabine, docetaxel, doxorubicin, elmustin, erlotinib, etoposide, gemcitabine, idarubicin, imatinib, lomustine, methotrexate, mitomycin, mitoxantrone, oxaliplatin, paclitaxel, pemetrexed, sunitinib, topotecan, treosulfan, vemurafenib, vinblastine, vincristine, vindesine and/or vinorelbine were selected for this study. The cell lines selected include PAXF 546, PAXF 1986, PACF 1998, PACF 2005, PAXF 2035, PAXF 2059, PAXF CAPAN-2, PAXF HPAC, and PACF PANC-1. These cells lines were treated with EndoTAG®-1 (cationic liposomal paclitaxel formulation) and empty cationic liposomes (used for control), which were manufactured according to the method of EndoTAG®-1.

One vial of EndoTAG-1® containing 6.4 mg paclitaxel was dissolved in 23 mL water for injection by gentle shaking for 20 times until no undissolved powder is observed. The vial was stored at room temperature for at least 30 minutes to allow for complete reconstitution. The vial was repeatedly shaken after the storage period. Temperature did not exceed

30°C at any time.

Stock solutions of EndoTAG®-1 and empty liposomes, each containing 300 μM in 10.5% trehalose were prepared right before adding to the assay wells. In a first step 1:2 dilution was prepared to reach nominal paclitaxel concentration of 128 mg/L. EndoTAG-1® and empty liposomes were next serially diluted in half-log steps with 10.5% trehalose on an intermediate dilution plate, followed by a further 1:10 dilution with 10.5% trehalose. Finally, 10 μL taken from this dilution plate was transferred to 140 μL/well of the cell culture plate. EndoTAG-1® and empty liposomes were tested at 10 concentrations in triplicate in half-log steps up to 1 μM. Thus, final concentration of trehalose in each assay well was 0.7% w/v.

Cell lines were routinely passaged once or twice weekly and maintained in culture for up to 20 passages. Cells were grown at 37°C in a humidified atmosphere with 5% $CO_2$ in RPMI 1640 medium (25 mM HEPES, with L-glutamine, #FG1385, Biochrom, Berlin, Germany) supplemented with 10% (v/v) fetal calf serum (Sigma, Taufkirchen, Germany) and 0.1 mg/mL gentamicin (Life Technologies, Karlsruhe, Germany).

The CellTiter-Blue® Cell Viability Assay (#G8081, Promega) was used to investigate anti-tumor activity. Cells were harvested from exponential phase cultures, counted and plated in 96-well flat-bottom microtiter plates at a cell density of 8,000 - 12,000 cells/well depending on the cell line's growth rate. After a 24 h recovery period to allow the cells to resume exponential growth, 10 μL of culture medium (four control wells/plate) or of culture medium with EndoTAG®-1 or culture medium with empty liposomes was added to the cells. EndoTAG®-1 was applied at 10 concentrations in triplicate in half-log increments up to 1 μM and treatment continued for three days. After treatment of cells, 20 μL/well CellTiter-Blue® reagent was added. Following an incubation period of up to four hours, fluorescence (FU) was measured by using the Enspire Multimode Plate Reader (excitation λ= 531 nm, emission λ= 615 nm). For calculations, the mean values of triplicate data were used. Pancreatic cancer cell lines resistant to one or more antineoplastic agents (see Table 2) were treated with EndoTAG®-1 and empty liposomes. $IC_{50}$ of EndoTAG®-1 are shown in Table 1.

Table 1 shows that EndoTAG®-1 is effective in inhibiting the growth of eight MDR pancreatic cells. No growth inhibitory effect was observed with MDR pancreatic cell lines treated with empty liposomes.

Table 1 Relative $IC_{50}$ (μM)

| Cell line \ treatment | EndoTAG-1 | Empty liposomes |
|---|---|---|
| PAXF 546 | 0.005 | 1.0 |
| PAXF 1986 | 0.011 | 1.0 |
| PAXF 1998 | 0.039 | 1.0 |
| PAXF 2005 | 0.006 | 1.0 |
| PAXF 2035 | 0.032 | 1.0 |
| PAXF 2059 | 0.004 | 1.0 |
| PAXF CAPAN-2 | 0.013 | 1.0 |
| PAXF PANC-1 | 0.008 | 1.0 |

Table 2 Cell lines exhibiting drug resistance ($IC_{50}$) (μM)

| Drug \ Cell line | PAXF CAPAN-2 | PAXF HPAC | PAXF PANC-1 | PAXF 546 | PAXF 1986 | PAXF 1998 | PAXF 2005 | PAXF 2035 | PAXF 2059 |
|---|---|---|---|---|---|---|---|---|---|
| 5-Fluorouracil | | | 3.228 | 1.246 | 2.253 | 7.475 | 10.328 | 105.27 | |
| Bleomycin sulfate | | | 0.149 | 10.705 | | | | | |
| Bortezomib | | | 0.018 | 0.005 | | | | | |
| Carboplatin | | | 15.634 | 17.082 | | | | | |
| Cisplatin | 62.143 | 31.722 | 7.441 | 11.513 | 4.831 | 75.726 | 10.768 | 46.021 | 40.966 |
| Cytarabine | | | 0.158 | 4.637 | | | | | |
| Docetaxel | | | 0.000 | 0.104 | 0.006 | 0.015 | 0.100 | 0.004 | 0.100 |
| Doxorubicin HCl | | | 0.049 | 0.134 | 0.059 | 1.232 | 0.251 | 0.795 | 0.131 |
| Elmustin | | | 374.97 | 722.40 | | | | | |
| Erlotinib HCl | | | 113.26 | 46.123 | 100.00 | 99.090 | 52.700 | 83.616 | |
| Etoposide | | | 1.496 | 7.405 | | | | | |
| Gemcitabine HCl | 3.670 | 0.421 | 5.216 | 0.097 | 0.008 | 0.158 | 0.390 | 1.492 | 0.015 |
| Idarubicin | | | 0.012 | 0.131 | | | | | |
| Imatinib, mesylate | | | 36.767 | 20.052 | | | | | |
| Lomustine | | | 43.617 | 142.17 | | | | | |
| Methotrexate | | | 10.000 | 0.066 | | | | | |
| Methotrexate Hydrat | | | 0.038 | 0.129 | | 0.160 | 10.000 | | 0.031 |
| Mitomycin C | | | 1.121 | 0.465 | 0.188 | 0.987 | 0.276 | 0.678 | |
| Mitoxantron 2HCl | | | 0.010 | 0.187 | 0.035 | 0.240 | 0.016 | 0.067 | 0.035 |
| Oxaliplatin | | | 0.994 | 1.240 | 1.606 | 34.622 | 12.475 | 99.179 | |
| Paclitaxel | 0.444 | 0.383 | 0.046 | 0.441 | 0.204 | 0.117 | 0.312 | 0.144 | 0.261 |
| Pemetrexed, dinatrium | | | 12.985 | 0.114 | | | | | |
| Sunitinib malate | | | 8.551 | 7.381 | 4.236 | 13.216 | 7.093 | 2.404 | 16.472 |
| Topotecan HCl | | | 0.042 | 0.080 | | | | | |
| Treosulfan | | | 2.015 | 48.058 | | | | | |
| Vemurafenib | | | 16.078 | 5.329 | 9.337 | 10.768 | 6.753 | 11.432 | 11.392 |
| Vinblastine sulfate | | | 0.001 | 0.071 | | | | | |
| Vincristine sulfate | | | 0.003 | 0.247 | 0.300 | 0.008 | 0.045 | 0.462 | |
| Vindesin sulfate | | | 0.005 | 0.109 | | | | | |
| Vinorelbine bistartrate | | | 0.002 | 0.072 | | | | | |

Example 2: EndoTAG®-1 plus gemcitabine vs. gemcitabine monotherapy in patients with locally advanced and/or metastatic adenocarcinoma of the pancreas

1.1 Objectives

The objective of the study is to assess the safety, efficacy and quality of life of a combination therapy of EndoTAG®-1 plus gemcitabine vs. gemcitabine monotherapy in patients with locally advanced and/or metastatic adenocarcinoma of the pancreas eligible for second-line therapy after failing first-line therapy with FOLFIRINOX.

1.2 Endpoints

Primary Efficacy Endpoint:

Overall survival time is defined as time from randomization to death from any cause or last day known to be alive.

Secondary Efficacy Endpoints:

1. Progression Free Survival (PFS)
Progression Free Survival time is defined as the time from randomization to either first observation of progressive disease or occurrence of death.
2. Percentage of subjects with Objective Response (OR) according to Response Evaluation Criteria in Solid Tumors Version 1.1 (RECIST v.1.1)
Percentage of subjects with objective response is based on assessment of complete response (CR) or partial response (PR) according to RECIST v.1.1.
3. Duration of Response (DR)
Duration of Response is defined as the time from the first documentation of objective tumor response (date of the first CR or PR) to objective tumor progression or death due to any cause.
4. Percentage of subjects with disease control according to RECIST v.1.1
Percentage of subjects with disease control is based on assessment of complete response (CR) or partial response (PR) or stable disease (SD) according to RECIST v.1.1

5. Change from baseline in Quality of Life (QoL) scale
6. Changes from baseline in ECOG performance status
Eastern Cooperative Oncology Group (ECOG) performance status is used to quantify the functional status of subjects. Number of patients with improvement, steady state, and deterioration at the end of cycle 1 (or at the end of full treatment course) will be evaluated.
7. Pain intensity using a Visual Analog Scale (VAS)
The VAS is used to assess pain intensity. The score can vary between "0" and "10" wherein, 0 = no pain and 10 = the worst possible pain.
8. Serum Carcinoma Antigen 19-9 (CA 19-9) response rate
Responders are defined as subjects with a reduction in CA 19-9 levels by least 50% from baseline to the end of cycle 1 (or end of full treatment course).

Safety Endpoints

1. Incidence and percentage of subjects with treatment-emergent adverse events (TEAEs) during cycle 1 and the full treatment course
2. AEs leading to discontinuation of study medication, interruption of infusion of gemcitabine or EndoTAG®-1, or postponement of subsequent dose of study medication
3. Incidence and percentage of clinically significant abnormal laboratory values during cycle 1 and the full treatment course
4. Incidence and percentage of clinically significant abnormal physical examination and vital signs during cycle 1 and the full treatment course

1.3 Study Design

This is a randomized controlled, open label phase-3 study to evaluate the safety and efficacy of a combination regimen of twice weekly infusions of EndoTAG®-1 (Lipid Complexed Paclitaxel) with weekly administration of gemcitabine compared with gemcitabine monotherapy in subjects with measurable locally advanced and/or metastatic adenocarcinoma of the pancreas who are eligible for second-line therapy after failing first-line therapy with FOLFIRINOX.
Eligible subjects will be randomized to one of the two treatment arms :

- Arm A: Treatment with EndoTAG®-1 22 mg/m$^2$ twice weekly plus gemcitabine 1000 mg/m$^2$ once weekly, for 1 cycle consisting of 7 weeks and 1 week rest followed by subsequent cycles consisting of 3 weeks of treatment and 1 week rest until any one of the following occurs: progressive disease or unacceptable toxicity or withdrawal of patient consent.
- Arm B: Treatment with gemcitabine 1000 mg/m$^2$ once weekly, for 1 cycle consisting of 7 weeks and 1 week rest followed by subsequent cycles consisting of 3 weeks of treatment and 1 week rest until any one of the following occurs: progressive disease or unacceptable toxicity or withdrawal of patient consent.

The randomization is stratified by

- Subjects with locally advanced vs metastatic pancreatic cancer
- Subjects with ECOG performance status 0 vs 1

The first treatment cycle is last at least 8 weeks and include 7 weekly (Arm B) or 14 twice weekly (Arm A) treatment visits followed by an EoT visit. Subjects may continue to receive additional cycles of therapy until progressive disease or intolerable toxicity as per clinical judgment of the Investigator.
Tumor response according to RECIST (version 1.1; Eisenhauer et al. 2009) is evaluated on a scheduled basis every 8 weeks (±3 days) from randomization (regardless of the timing of treatment cycles) until disease progression is documented or until the cut-off date of the study, whichever comes earlier. Subjects are monitored regularly for safety parameters, pain and quality of life.
After completing treatment, subjects who diagnosed with progressive disease (PD) are attend up to 6 Follow-up Visits every 8 weeks for 48 weeks, following which subjects will be followed-up by telephone every 8 weeks for survival. Subjects who experienced PD during Treatment Phase are undergo only one safety follow-up visit (4-8 weeks after the EoT visit), and then enter phone follow-up directly. Follow-up visits will be performed for the evaluation of survival status, safety parameters, QoL/pain and administration of other anti-tumor treatment until death or end of the study, whichever

comes first.

Anti-tumor therapy after termination of study treatment is at the discretion of the Investigator. However, the OFF regimen (O = Oxaliplatin; F = Fluorouracil; F = Leucovorin Calcium (Folinic Acid)) is recommended.

The cut-off date for the main analysis is 12 months after the last subject is randomized or the last subject alive has been followed up for at least 12 months, whatever applies first. Subjects being still under treatment with study medication at this cut-off date will enter the extension phase of this trial. These subjects are followed up until 28 days after the last administration of study medication.

1.4 Selection of study population

Inclusion Criteria

Potential subjects are required to meet all of the following criteria for enrollment into the study and subsequent randomization:

1.

$$\text{Age} \geq 18 \text{ years}$$

2. Written informed consent
3. Histologically or cytologically confirmed adenocarcinoma of the pancreas
4. Metastatic or locally advanced disease that is considered unresectable
5. Measurable / assessable disease according to RECIST v.1.1
6. Documented disease progression on first line FOLFIRINOX
7. Negative pregnancy test
8. Willingness to perform double-barrier contraception during study and for 4 weeks after last treatment
9. ECOG performance status 0 or 1

Exclusion Criteria

Patients who meet one or more of the following criteria cannot be considered eligible to participate in the study:

1. Cardiovascular disease, New York Heart Association (NYHA) III or IV
2. History of severe supraventricular or ventricular arrhythmia
3. History of coagulation or bleeding disorder
4. History of acute myocardial infarction within 6 months before randomization
5. History of congestive heart failure
6. Acute or chronic inflammation (autoimmune or infectious)
7. Significant active/unstable non-malignant disease likely to interfere with study assessments
8. Laboratory tests (hematology, chemistry) outside specified limits:

a)

$$\text{WBC} \leq 3 \text{ x } 10^3/\text{mm}^3$$

b)

$$\text{ANC} \leq 1.5 \text{ x } 10^3/\text{mm}^3$$

c)

$$\text{Platelets} \leq 100.000/\text{mm}^3$$

d)

$$\text{Hb} \leq 9.0 \text{ g/dl } (\leq 5.6 \text{ mmol/l})$$

e)

PTT > 1.5 x ULN specified limits:

f)

Serum creatinine > 2.0 mg/dl (> 176.8 μmol/l)

g) AST and/or ALT > 2.5 x ULN; for patients with significant liver metastasis AST and/or ALT > 5 x ULN

h)

Alkaline phosphatase > 2.5 x ULN

i)

Total bilirubin > 2 x ULN

j)

Albumin < 2.5 g/dL

9. Clinically significant ascites

10.

Immunotherapy < 6 weeks prior to enrollment.

11. Any anti-tumor treatment (except FOLFIRINOX as the first-line therapy) for pancreatic adenocarcinoma before enrollment. Note: Subjects who have undergone surgical interventions for pancreatic adenocarcinoma will be eligible.

12. Any radiotherapy for pancreatic adenocarcinoma before enrollment except for treatment of bone metastases if target lesions are not included in the irradiated field

13.

Major surgery < 4 weeks prior to enrollment

14. Pregnant or nursing

15.

Investigational medicinal product < 4 weeks of enrollment

16. Documented HIV history

17. Active hepatitis B or hepatitis C

18. Known hypersensitivity to any component of the EndoTAG®-1 and/or gemcitabine formulations

19. History of malignancy other than pancreatic cancer < 3 years prior to enrollment, except non-melanoma skin cancer or carcinoma in situ of the cervix treated locally

20. Vulnerable populations (e.g. subjects unable to understand and give voluntary informed consent)

2.5 Drug administration

Arm A:

Treatment cycle 1: EndoTAG®-1 is given at a dose of 22 mg/m$^2$ as an intravenous infusion which should be started slowly and increased to a maximum of 1.5 ml/min (15 min at 0.5 ml/min, 15 min at 1.0 ml/min. and thereafter 1.5 ml/min.) on days 1, 4, 8, 11, 15, 18, 22, 25, 29, 32, 36, 39, 43 and 46 plus gemcitabine 1000 mg/m$^2$, 30 min. i.v. infusion on days 4, 11, 18, 25, 32, 39, and 46 of cycle 1 until any one of the following occurs: progressive disease or unacceptable toxicity or withdrawal of patient consent

Subsequent treatment cycles: EndoTAG®-1 on days 1, 4, 8, 11, 15, and 18 plus gemcitabine on days 4, 11, and 18

of all subsequent cycles, until any one of the following occurs: progressive disease or unacceptable toxicity or withdrawal of patient consent

Arm B:

Treatment cycle 1: Gemcitabine 1000 mg/m$^2$, 30 min. i.v. infusion on days 4, 11, 18, 25, 32, 39, and 46 of cycle 1 until any one of the following occurs: progressive disease or unacceptable toxicity or withdrawal of patient consent
Subsequent treatment cycles: Gemcitabine on days 4, 11, and 18 of all subsequent cycles, until any one of the following occurs: progressive disease or unacceptable toxicity or withdrawal of patient consent
2.6 Dose adjustment in the event of toxicities:
The doses and timing of treatment is modified based on toxicities experienced by the patient. Dose modification and retreatment are outlined below:
Dose Modifications for EndoTAG®-1:
Criteria for Dose Modifications

- Grade 4 neutropenia lasting 7 or more days
- Febrile neutropenia
- Grade 4 thrombocytopenia
- Grade 3 thrombocytopenia with significant bleeding or requiring transfusion
- Grade $\geq$ 3 stomatitis/vomiting/diarrhea
- Other ≥ Grade 3 and 4 toxicities (Except Grade 3 fatigue/asthenia or transient arthralgia/myalgia for which no dose modification is required.)

If any of the above mentioned toxicity criteria are present, no study medication is administered at this visit. If the toxicity criteria are no longer fulfilled at the next scheduled visit, EndoTAG®-1 is administered at a reduced dose of 11 mg/m$^2$. If the subject tolerates treatment at the reduced dose (i.e. does not develop any of the above mentioned toxicities), EndoTAG®-1 dose should be re-escalated to 22 mg/m$^2$. If re-escalation is not tolerated by the subject, the dose is permanently reduced to 11 mg/m$^2$. The attempt for re-escalation of the EndoTAG-1 dose is made only once throughout the study.

Dose Modifications for Gemcitabine:

Dose Modifications for Hematologic Adverse Reactions

| Absolute granulocyte count (x $10^6$/L) | | Platelet count (x $10^6$/L) | % of full dose |
|---|---|---|---|
| ≥ 1000 | And | ≥ 100,000 | 100% |
| 500-999 | Or | 50,000-99,999 | 75% |
| <500 | Or | < 50,000 | Hold |

Dose Modifications for Non-Hematologic Adverse Reactions

Permanently discontinue Gemcitabine for any of the following:

- Unexplained dyspnea or other evidence of severe pulmonary toxicity
- Severe hepatic toxicity
- Hemolytic-uremic syndrome
- Capillary leak syndrome
- Posterior reversible encephalopathy syndrome

Withhold gemcitabine or reduce dose by 50% for other severe (Grade 3 or 4) non-hematological toxicity until resolved.

## Claims

1. A cationic liposomal formulation comprising one or more cationic lipids and a therapeutically effective amount of paclitaxel for use:

   (a) in a method of treating refractory or resistant pancreatic cancer, wherein the method comprises administering to a subject in need thereof a cationic liposomal formulation comprising one or more cationic lipids and a therapeutically effective amount of paclitaxel
   wherein the refractory or resistant pancreatic cancer is refractory or resistant to:

   (i) one or more antineoplastic agents comprising fluorouracil, bleomycin, bortezomib, carboplatin, cisplatin, cytarabine, docetaxel, doxorubicin, elmustin, erlotinib, etoposide, gemcitabine, idarubicin, imatinib, lomustine, methotrexate, mitomycin, mitoxantrone, oxaliplatin, paclitaxel, pemetrexed, sunitinib, topotecan, treosulfan, vemurafenib, vinblastine, vincristine, vindesine, or vinorelbine,
   (ii) a fluorouracil-based combination therapy, particularly a combination of oxaliplatin, leucovorin, irinotecan, and fluorouracil,
   (iii) a gemcitabine-based combination therapy,
   (iv) a growth factor inhibitor, particularly wherein the growth factor inhibitor is selected from a group consisting of erlotinib, cetuximab, gefinitib, imatinib, panitumumab, sunitinib, and vemurafenib, or
   (v) an antimitotic agent, particularly wherein the antimitotic agent is selected from a group consisting of paclitaxel, docetaxel, vinblastine, vincristine, vindesine, and vinorelbine, or

   (b) in a method for *in vivo* inhibiting the growth of multidrug resistant (MDR) pancreatic cancer cells comprising administering to MDR pancreatic cells a cationic liposomal formulation comprising one or more cationic lipids and a therapeutically effective amount of paclitaxel.

2. The cationic liposomal formulation for use as in claim 1(a) wherein the method further comprises administering to a subject in need thereof a therapeutically effective amount of gemcitabine.

3. The cationic liposomal formulation for use as in claim 1(a)(ii), wherein the subject has previously been treated with an intravenous infusion of 70 mg/m$^2$ to 100 mg/m$^2$ oxaliplatin followed by an intravenous infusion of 300 mg/m$^2$ to 500 mg/m$^2$ leucovorin concomitantly with an intravenous infusion of 90 mg/m$^2$ to 270 mg/m$^2$ irinotecan, followed by an intravenous bolus of 300 mg/m$^2$ to 800 mg/m$^2$ fluorouracil and an intravenous infusion of 1200 mg/m$^2$ to 3600 mg/m$^2$ fluorouracil, particularly wherein the subject has previously been treated with an intravenous infusion of 85 mg/m$^2$ oxaliplatin followed by an intravenous infusion of 400 mg/m$^2$ leucovorin concomitantly with an intravenous infusion of 180 mg/m$^2$ irinotecan, followed by an intravenous bolus of 400 mg/m$^2$ fluorouracil and an intravenous infusion of 2400 mg/m$^2$ fluorouracil.

4. The cationic liposomal formulation for use as in claim 1(a)(i), wherein the subject has previously been treated with one or more antineoplastic agents without administering a pulsed dose, particularly wherein the subject has previously been treated with an intravenous infusion of 70 mg/m$^2$ to 100 mg/m$^2$ oxaliplatin followed by an intravenous infusion of 300 mg/m$^2$ to 500 mg/m$^2$ leucovorin concomitantly with an intravenous infusion of 90 mg/m$^2$ to 180 mg/m$^2$ irinotecan, followed by an intravenous infusion of 1200 mg/m$^2$ to 3600 mg/m$^2$ fluorouracil, more particularly wherein the subject has previously been treated with an intravenous infusion of 85 mg/m$^2$ oxaliplatin followed by an intravenous infusion of 400 mg/m$^2$ leucovorin concomitantly with an intravenous infusion of 130 mg/m$^2$ to 150 mg/m$^2$ irinotecan, followed by an intravenous infusion of 2400 mg/m$^2$ fluorouracil.

5. The cationic liposomal formulation for use as in any one of claims 2 to 4, wherein the method comprises administering 1 mg/m$^2$ to 60 mg/m$^2$ paclitaxel in the cationic liposomal formulation and 300 mg/m$^2$ to 1500 mg/m$^2$ gemcitabine to the subject, particularly wherein the method comprises administering 11 mg/m$^2$ to 22 mg/m$^2$ paclitaxel in the cationic liposomal formulation and 500 mg/m$^2$ to 1000 mg/m$^2$ gemcitabine to the subject.

6. The cationic liposomal formulation for use as in any one of claims 2 to 4, wherein:

   (i) the cationic liposomal formulation is administered twice weekly, and gemcitabine is administered once weekly, or
   (ii) the cationic liposomal formulation is administered to the subject at a rate of 0.5 mL/min for first 15 minutes, followed by a rate of 1.0 mL/min for second 15 minutes, and followed by a rate of 1.5 mL/min after 30 minutes.

7. The cationic liposomal formulation for use as in claim 5, wherein the cationic liposomal formulation is administered on days 1, 4, 8, 11, 15, 18, 22, 25, 29, 32, 36, 39, 43, and 46 and gemcitabine is administered on days 4, 11, 18, 25, 32, 39 and 46 of a first treatment cycle of seven weeks, particularly wherein the first treatment cycle is followed by one or more subsequent treatment cycles, the cationic liposomal formulation is administered on days 1, 4, 8, 11, 15, and 18 and gemcitabine is administered on days 4, 11, and 18 of a subsequent treatment cycle of three weeks, and a dosing interval between a first treatment cycle and a subsequent treatment cycle or between two subsequent treatment cycles is one week.

8. The cationic liposomal formulation for use as in any one of claims 1(a) and 2 to 4, wherein the cationic liposomal formulation comprises:

(i) a cationic lipid from 30 mole% to 99.9 mole%, paclitaxel in an amount of at least 0.1 mole% and a neutral or an anionic lipid in an amount of 30 mole% to 55 mole%, and the cationic liposomal formulation has a positive zeta potential in 0.05 M KCl solution at pH 7.5 at room temperature,
(ii) DOTAP, DOPC, and paclitaxel, or
(iii) DOTAP, DOPC, and paclitaxel in a mole ratio of 50:47:3, or
(iv) wherein the cationic lipid is N-[1-(2,3-dioleoyloxy)propyl]-N,N,N-trimethyl ammonium salt (DOTAP); dimethyldioctadecyl ammonium bromide (DDAB); 1,2-diacyloxy-3-trimethylammonium propane N-[1-(2,3-dioloyloxy) propyl]-N, N-dimethyl amine (DODAP); 1,2-diacyloxy-3-dimethylammonium propane; N-[1-(2,3-dioleyloxy)propyl]-N,N,N-trimethylammonium chloride (DOTMA); 1,2-dialkyloxy-3-dimethylammonium propane; dioctadecylamidoglycylspermine (DOGS); 3β-[N- (N',N'-dimethylamino-ethane)carbamoyl]cholesterol (DC-Chol); 2, 3-dioleoyloxy-N-(2-(sperminecarboxamido)-ethyl)-N, N-dimethyl-1-propanaminium trifluoroacetate (DOSPA); β-alanyl cholesterol; cetyl trimethyl ammonium bromide (CTAB); diC14-amidine; N-tert-butyl-N'-tetradecyl-3-tetradecylamino-propionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethylN,N'-bis(2-hydroxylethyl)-2,3-dioleoyloxy-1,4-butanediammonium iodide; 1-[2- (acyloxy)ethyl]2-alkyl (alkenyl)-3-(2-hydroxyethyl)-imidazolinium chloride; 1,2- dioleoyl-3-dimethyl-hydroxyethylammonium bromide (DORI); 1,2-dioleyloxypropyl3-dimethylhydroxyethylammonium bromide (DORIE); 1,2-dioleyloxypropyl-3-dimethylhydroxypropylammonium bromide (DORIE-HP); 1,2-dioleyloxypropyl-3-dimethylhydroxybutylammonium bromide (DORIE-HS); 1,2-dioleyloxypropyl-3-dimethylhydroxypentylammonium bromide (DORIE-Hpe); 1,2-dimyristyloxypropyl3-dimethylhydroxylethylammonium bromide (DMRIE); 1,2-dipalmityloxypropyl-3-dimethylhydroxyethylammonium bromide (DPRIE); 1,2-disteryloxypropyl-3-dimethylhydroxyethylammonium bromide (DSRIE); or 1,2-diacyl-sn-glycerol-3-ethylphosphocholine, particularly wherein the 1-[2-(acyloxy)ethyl]2-alkyl (alkenyl)-3-(2-hydroxyethyl)- imidazolinium chloride is 1-[2-(9(Z)-octadecenoyloxy)ethyl]-2-(8(Z)-heptadecenyl3-(2-hydroxyethyl)-imidazoliniumchloride (DOTIM) or 1-[2-(hexadecanoyloxy)ethyl]-2-pentadecyl-3-(2-hydroxyethyl)imidazolinium chloride (DPTIM).

9. The cationic liposomal formulation for use as in claim 8(i), wherein the neutral lipid is:

(a) cholesterol, phospholipid, lysolipid, sphingolipid, or pegylated lipid with a neutral charge,
(b) lysophospholipid, or
(c) 1,2-diacyl-sn-glycero-3-phosphoethanolamine, 1,2-diacyl-sn-glycero-3-phosphocholine, or sphingomyelin, particularly wherein 1,2-diacyl-sn-glycero-3-phosphoethanolamine is 1,2-dioleoyl-sn-glycero-3-phosphoethanolamine (DOPE) or 1,2-dioleoyl-sn-glycero-3-phosphocholine (DOPC).

10. The cationic liposomal formulation for use as in claim 2, wherein the cationic liposomal formulation and the gemcitabine are administered simultaneously but separately or are administered sequentially.

11. The cationic liposomal formulation for use as in claim 1(b), wherein the method further comprises administering a therapeutically effective amount of gemcitabine, particularly wherein the cationic liposomal formulation and the therapeutically effective amount of gemcitabine are administered simultaneously, but separately, or are administered sequentially.

12. The cationic liposomal formulation for use as in claim 1(a) or 2, wherein the method is preceded by a neoadjuvant therapy.

## Patentansprüche

1. Kationische liposomale Formulierung, umfassend ein oder mehrere kationische Lipide und eine therapeutisch wirksame Menge Paclitaxel zur Verwendung:

   (a) in einem Verfahren zur Behandlung von refraktärem oder resistentem Pankreaskrebs, wobei das Verfahren Verabreichen einer kationischen liposomalen Formulierung, umfassend ein oder mehrere kationische Lipide und eine therapeutisch wirksame Menge Paclitaxel, an ein Individuum, welches dessen bedarf, umfasst,
   wobei der refraktäre oder resistente Pankreaskrebs refraktär oder resistent gegenüber:

   (i) einem oder mehreren antineoplastischen Mitteln, umfassend Fluorouracil, Bleomycin, Bortezomib, Carboplatin, Cisplatin, Cytarabin, Docetaxel, Doxorubicin, Elmustin, Erlotinib, Etoposid, Gemcitabin, Idarubicin, Imatinib, Lomustin, Methotrexat, Mitomycin, Mitoxantron, Oxaliplatin, Paclitaxel, Pemetrexed, Sunitinib, Topotecan, Treosulfan, Vemurafenib, Vinblastin, Vincristin, Vindesin oder Vinorelbin,
   (ii) einer Fluorouracil-basierten Kombinationstherapie, insbesondere einer Kombination aus Oxaliplatin, Leucovorin, Irinotecan und Fluorouracil,
   (iii) einer Gemcitabin-basierten Kombinationstherapie,
   (iv) einem Wachstumsfaktorinhibitor, insbesondere wobei der Wachstumsfaktorinhibitor ausgewählt ist aus einer Gruppe, bestehend aus Erlotinib, Cetuximab, Gefitinib, Imatinib, Panitumumab, Sunitinib und Vemurafenib, oder
   (v) einem antimitotischen Mittel, insbesondere wobei das antimitotische Mittel ausgewählt ist aus einer Gruppe, bestehend aus Paclitaxel, Docetaxel, Vinblastin, Vincristin, Vindesin und Vinorelbin, ist, oder

   (b) in einem Verfahren zum In-vivo-Inhibieren des Wachstums von multiarzneimittelresistenten (MDR) Pankreaskrebszellen, umfassend Verabreichen einer kationischen liposomalen Formulierung, umfassend ein oder mehrere kationische Lipide und eine therapeutisch wirksame Menge Paclitaxel, an MDR-Pankreaskrebszellen.

2. Kationische liposomale Formulierung zur Verwendung nach Anspruch 1(a), wobei das Verfahren ferner Verabreichen einer therapeutisch wirksamen Menge Gemcitabin an ein Individuum, welches dessen bedarf, umfasst.

3. Kationische liposomale Formulierung zur Verwendung nach Anspruch 1(a)(ii), wobei das Individuum zuvor mit einer intravenösen Infusion von 70 mg/m$^2$ bis 100 mg/m$^2$ Oxaliplatin, gefolgt von einer intravenösen Infusion von 300 mg/m$^2$ bis 500 mg/m$^2$ Leucovorin gleichzeitig mit einer intravenösen Infusion von 90 mg/m$^2$ bis 270 mg/m$^2$ Irinotecan, gefolgt von einem intravenösen Bolus von 300 mg/m$^2$ bis 800 mg/m$^2$ Fluorouracil und einer intravenösen Infusion von 1200 mg/m$^2$ bis 3600 mg/m$^2$ Fluorouracil behandelt worden ist, insbesondere wobei das Individuum zuvor mit einer intravenösen Infusion von 85 mg/m$^2$ Oxaliplatin, gefolgt von einer intravenösen Infusion von 400 mg/m$^2$ Leucovorin gleichzeitig mit einer intravenösen Infusion von 180 mg/m$^2$ Irinotecan, gefolgt von einem intravenösen Bolus von 400 mg/m$^2$ Fluorouracil und einer intravenösen Infusion von 2400 mg/m$^2$ Fluorouracil behandelt worden ist.

4. Kationische liposomale Formulierung zur Verwendung nach Anspruch 1(a)(i), wobei das Individuum zuvor mit einem oder mehreren antineoplastischen Mitteln ohne Verabreichen einer Pulsdosis behandelt worden ist, insbesondere wobei das Individuum zuvor mit einer intravenösen Infusion von 70 mg/m$^2$ bis 100 mg/m$^2$ Oxaliplatin, gefolgt von einer intravenösen Infusion von 300 mg/m$^2$ bis 500 mg/m$^2$ Leucovorin gleichzeitig mit einer intravenösen Infusion von 90 mg/m$^2$ bis 180 mg/m$^2$ Irinotecan, gefolgt von einer intravenösen Infusion von 1200 mg/m$^2$ bis 3600 mg/m$^2$ Fluorouracil behandelt worden ist, weiterhin insbesondere wobei das Individuum zuvor mit einer intravenösen Infusion von 85 mg/m$^2$ Oxaliplatin, gefolgt von einer intravenösen Infusion von 400 mg/m$^2$ Leucovorin gleichzeitig mit einer intravenösen Infusion von 130 mg/m$^2$ bis 150 mg/m$^2$ Irinotecan, gefolgt von einer intravenösen Infusion von 2400 mg/m$^2$ Fluorouracil behandelt worden ist.

5. Kationische liposomale Formulierung zur Verwendung nach einem der Ansprüche 2 bis 4, wobei das Verfahren Verabreichen von 1 mg/m$^2$ bis 60 mg/m$^2$ Paclitaxel in der kationischen liposomalen Formulierung und von 300 mg/m$^2$ bis 1500 mg/m$^2$ Gemcitabin an das Individuum umfasst, insbesondere wobei das Verfahren Verabreichen von 11 mg/m$^2$ bis 22 mg/m$^2$ Paclitaxel in der kationischen liposomalen Formulierung und von 500 mg/m$^2$ bis 1000 mg/m$^2$ Gemcitabin an das Individuum umfasst.

6. Kationische liposomale Formulierung zur Verwendung nach einem der Ansprüche 2 bis 4, wobei:

   (i) die kationische liposomale Formulierung zweimal wöchentlich verabreicht wird und Gemcitabin einmal

wöchentlich verabreicht wird, oder
(ii) die kationische liposomale Formulierung dem Individuum mit einer Rate von 0,5 mL/min für erste 15 Minuten, gefolgt von einer Rate von 1,0 mL/min für zweite 15 Minuten, und gefolgt von einer Rate von 1,5 mL/min nach 30 Minuten verabreicht wird.

**7.** Kationische liposomale Formulierung zur Verwendung nach Anspruch 5, wobei die kationische liposomale Formulierung an Tagen 1, 4, 8, 11, 15, 18, 22, 25, 29, 32, 36, 39, 43 verabreicht wird und 46 und Gemcitabin an Tagen 4, 11, 18, 25, 32, 39 und 46 eines ersten Behandlungszyklus von sieben Wochen verabreicht wird, insbesondere wobei auf den ersten Behandlungszyklus ein oder mehrere nachfolgende Behandlungszyklen folgen, die kationische liposomale Formulierung an Tagen 1, 4, 8, 11, 15 und 18 und Gemcitabin an Tagen 4, 11 und 18 eines nachfolgenden Behandlungszyklus von drei Wochen verabreicht wird, und ein Dosierungsintervall zwischen einem ersten Behandlungszyklus und einem nachfolgenden Behandlungszyklus oder zwischen zwei nachfolgenden Behandlungszyklen eine Woche beträgt.

**8.** Kationische liposomale Formulierung zur Verwendung nach einem der Ansprüche 1(a) und 2 bis 4, wobei die kationische liposomale Formulierung umfasst:

(i) ein kationisches Lipid von 30 Mol-% bis 99,9 Mol-%, Paclitaxel in einer Menge von wenigstens 0,1 Mol-% und ein neutrales oder anionisches Lipid in einer Menge von 30 Mol-% bis 55 Mol-%, und die kationische liposomale Formulierung ein positives Zeta-Potential in 0,05 M KCl-Lösung bei pH 7,5 bei Raumtemperatur aufweist,
(ii) DOTAP, DOPC und Paclitaxel, oder
(iii) DOTAP, DOPC und Paclitaxel in einem Molverhältnis von 50:47:3, oder
(iv) wobei das kationische Lipid N-[1-(2,3-Dioleoyloxy)propyl]-N,N,N-trimethylammoniumsalz (DOTAP); Dimethyldioctadecylammoniumbromid (DDAB); 1,2-Diacyloxy-3-trimethylammoniumpropan; N-[1-(2,3-Dioleoyloxy) propyl]-N,N-dimethylamin (DODAP); 1,2-Diacylhoxy-3-dimethylammoniumpropan; N-[1-(2,3-Dioleoyloxy)propyl]-N,N,N-trimethylammoniumchlorid (DOTMA); 1,2-Dialkyloxy-3-dimethylammoniumpropan; Dioctadecylamidoglycylspermin (DOGS); 3β-[N-(N',N'-Dimethylamino-ethan)carbamoyl]cholesterol (DC-Chol); 2,3-Dioleoyloxy-N-(2-(Spermincarboxamido)-ethyl)-N,N-dimethyl-1-propanaminiumtrifluoracetat (DOSPA); β-Alanylcholesterol; Cetyltrimethylammoniumbromid (CTAB); diC14-Amidin; N-tert-Butyl-N'-tetradecyl-3-tetradecylaminopropionamidin; 14Dea2; N-(Alpha-trimethylammonioacetyl)didodecyl-D-glutamatchlorid (TMAG); O,O'-Ditetradecanoyl-N-(trimethylammonioacetyl)diethanolaminchlorid; 1,3-Dioleoyloxy-2-(6-carboxy-spermyl)-propylamid (DOSPER); N,N,N',N'-Tetramethyl-N,N'-bis(2-hydroxyethyl)-2,3-dioleoyloxy-1,4-butandiammoniumiodid; 1-[2-(Acyloxy)ethyl]2-alkyl(alkenyl)-3-(2-hydroxyethyl)-imidazoliniumchlorid; 1,2-Dioleoyl-3-dimethyl-hydroxyethylammoniumbromid (DORI); 1,2-Dioleoyloxypropyl-3-dimethylhydroxyethylammoniumbromid (DORIE); 1,2-Dioleoyloxypropyl-3-dimethylhydroxypropylammoniumbromid (DORIE-HP); 1,2-Dioleoyloxypropyl-3-dimethylhydroxybutylammoniumbromid (DORIE-HS); 1,2-Dioleoyloxypropyl-3-dimethylhydroxyheptylammoniumbromid (DORIE-Hpe); 1,2-Dimyristyloxypropyl-3-dimethylhydroxyethylammoniumbromid (DMRIE); 1,2-Dipalmityloxypropyl-3-dimethylhydroxyethylammoniumbromid (DPRIE); 1,2-Disteryloxypropyl-3-dimethylhydroxyethylammoniumbromid (DSRIE); oder 1,2-Diacyl-sn-glycerol-3-ethylphosphocholin ist, insbesondere wobei das 1-[2-(Acyloxy)ethyl]2-alkyl(alkenyl)-3-(2-hydroxyethyl)-imidazoliniumchlorid 1-[2-(9(Z)-Octadecenyloxy) ethyl]-2-(8(Z)-Heptadecenyl)-3-(2-hydroxyethyl)imidazoliniumchlorid (DOTIM) oder 1-[2-(Hexadecanoyloxy) ethyl]-2-pentadecyl-3-(2-hydroxyethyl)imidazoliniumchlorid (DPTIM) ist.

**9.** Kationische liposomale Formulierung zur Verwendung nach Anspruch 8(i), wobei das neutrale Lipid:

(a) Cholesterol, Phospholipid, Lysolipid, Sphingolipid oder pegyliertes Lipid mit einer neutralen Ladung,
(b) Lysophospholipid, oder
(c) 1,2-Diacyl-sn-glycero-3-phosphoethanolamin, 1,2-Diacyl-sn-glycero-3-phosphocholin oder Sphingomyelin ist, insbesondere wobei 1,2-Diacyl-sn-glycero-3-phosphoethanolamin 1,2-Dioleoyl-sn-glycero-3-phosphoethanolamin (DOPE) oder 1,2-Dioleoyl-sn-glycero-3-phosphocholin (DOPC) ist.

**10.** Kationische liposomale Formulierung zur Verwendung nach Anspruch 2, wobei die kationische liposomale Formulierung und das Gemcitabin gleichzeitig, aber getrennt verabreicht werden oder sequenziell verabreicht werden.

**11.** Kationische liposomale Formulierung zur Verwendung nach Anspruch 1(b), wobei das Verfahren ferner Verabreichen einer therapeutisch wirksamen Menge Gemcitabin umfasst, insbesondere wobei die kationische liposomale Formulierung und die therapeutisch wirksame Menge Gemcitabin gleichzeitig, aber getrennt verabreicht werden oder sequenziell verabreicht werden.

**12.** Kationische liposomale Formulierung zur Verwendung nach Anspruch 1(a) oder 2, wobei dem Verfahren eine neoadjuvante Therapie vorausgeht.

## Revendications

**1.** Formulation liposomale cationique comprenant un ou plusieurs lipides cationiques et une quantité thérapeutiquement efficace de paclitaxel pour une utilisation :

(a) dans une méthode de traitement d'un cancer du pancréas réfractaire ou résistant, dans laquelle la méthode comprend l'administration à un sujet en ayant besoin d'une formulation liposomale cationique comprenant un ou plusieurs lipides cationiques et une quantité thérapeutiquement efficace de paclitaxel
dans laquelle le cancer du pancréas réfractaire ou résistant est réfractaire ou résistant à :

(i) un ou plusieurs agents antinéoplasiques comprenant le fluorouracile, la bléomycine, le bortézomib, le carboplatine, le cisplatine, la cytarabine, le docétaxel, la doxorubicine, l'elmustine, l'erlotinib, l'étoposide, la gemcitabine, l'idarubicine, l'imatinib, la lomustine, le méthotrexate, la mitomycine, la mitoxantrone, l'oxaliplatine, le paclitaxel, le pemetrexed, le sunitinib, le topotécan, le tréosulfan, le vemurafenib, la vinblastine, la vincristine, la vindésine ou la vinorelbine,
(ii) une thérapie combinée à base de fluorouracile, en particulier une combinaison d'oxaliplatine, de leucovorine, d'irinotécan et de fluorouracile,
(iii) une thérapie combinée à base de gemcitabine,
(iv) un inhibiteur de facteur de croissance, en particulier dans laquelle l'inhibiteur de facteur de croissance est sélectionné dans un groupe constitué de l'erlotinib, du cétuximab, du gefinitib, de l'imatinib, du panitumumab, du sunitinib et du vemurafenib, ou
(v) un agent antimitotique, en particulier dans laquelle l'agent antimitotique est sélectionné dans un groupe constitué du paclitaxel, du docétaxel, de la vinblastine, de la vincristine, de la vindésine et de la vinorelbine, ou

(b) dans une méthode pour inhiber in vivo la croissance de cellules de cancer du pancréas multirésistantes (MDR) comprenant l'administration à des cellules de cancer du pancréas MDR d'une formulation liposomale cationique comprenant un ou plusieurs lipides cationiques et une quantité thérapeutiquement efficace de paclitaxel.

**2.** La formulation liposomale cationique pour une utilisation selon la revendication 1(a), dans laquelle la méthode comprend en outre l'administration à un sujet en ayant besoin d'une quantité thérapeutiquement efficace de gemcitabine.

**3.** La formulation liposomale cationique pour une utilisation selon la revendication 1(a)(ii), dans laquelle le sujet a été préalablement traité par une perfusion intraveineuse de 70 mg/m$^2$ à 100 mg/m$^2$ d'oxaliplatine suivie d'une perfusion intraveineuse de 300 mg/m$^2$ à 500 mg/m$^2$ de leucovorine de manière concomitante avec une perfusion intraveineuse de 90 mg/m$^2$ à 270 mg/m$^2$ d'irinotécan, suivie d'un bolus intraveineux de 300 mg/m$^2$ à 800 mg/m$^2$ de fluorouracile et d'une perfusion intraveineuse de 1200 mg/m$^2$ à 3600 mg/m$^2$ de fluorouracile, en particulier dans laquelle le sujet a été préalablement traité par une perfusion intraveineuse de 85 mg/m$^2$ d'oxaliplatine suivie d'une perfusion intraveineuse de 400 mg/m$^2$ de leucovorine de manière concomitante avec une perfusion intraveineuse de 180 mg/m$^2$ d'irinotécan, suivie d'un bolus intraveineux de 400 mg/m$^2$ de fluorouracile et d'une perfusion intraveineuse de 2400 mg/m$^2$ de fluorouracile.

**4.** La formulation liposomale cationique pour une utilisation selon la revendication 1(a)(i), dans laquelle le sujet a été préalablement traité par un ou plusieurs agents antinéoplasiques sans administration d'une dose pulsée, en particulier dans laquelle le sujet a été préalablement traité par une perfusion intraveineuse de 70 mg/m$^2$ à 100 mg/m$^2$ d'oxaliplatine suivie d'une perfusion intraveineuse de 300 mg/m$^2$ à 500 mg/m$^2$ de leucovorine de manière concomitante avec une perfusion intraveineuse de 90 mg/m$^2$ à 180 mg/m$^2$ d'irinotécan, suivie d'une perfusion intraveineuse de 1200 mg/m$^2$ à 3600 mg/m$^2$ de fluorouracile, plus particulièrement dans laquelle le sujet a été préalablement traité par une perfusion intraveineuse de 85 mg/m$^2$ d'oxaliplatine suivie d'une perfusion intraveineuse de 400 mg/m$^2$ de leucovorine de manière concomitante avec une perfusion intraveineuse de 130 mg/m$^2$ à 150 mg/m$^2$ d'irinotécan, suivie d'une perfusion intraveineuse de 2400 mg/m$^2$ de fluorouracile.

**5.** La formulation liposomale cationique pour une utilisation selon l'une quelconque des revendications 2 à 4, dans

laquelle la méthode comprend l'administration de 1 mg/m$^2$ à 60 mg/m$^2$ de paclitaxel dans la formulation liposomale cationique et de 300 mg/m$^2$ à 1500 mg/m$^2$ de gemcitabine au sujet, en particulier dans laquelle la méthode comprend l'administration de 11 mg/m$^2$ à 22 mg/m$^2$ de paclitaxel dans la formulation liposomale cationique et de 500 mg/m$^2$ à 1000 mg/m$^2$ de gemcitabine au sujet.

**6.** La formulation liposomale cationique pour une utilisation selon l'une quelconque des revendications 2 à 4, dans laquelle :

(i) la formulation liposomale cationique est administrée deux fois par semaine, et la gemcitabine est administrée une fois par semaine, ou
(ii) la formulation liposomale cationique est administrée au sujet à un débit de 0,5 mL/min pendant les 15 premières minutes, suivi d'un débit de 1,0 mL/min pendant les 15 minutes suivantes, et suivi d'un débit de 1,5 mL/min après 30 minutes.

**7.** La formulation liposomale cationique pour une utilisation selon la revendication 5, dans laquelle la formulation liposomale cationique est administrée aux jours 1, 4, 8, 11, 15, 18, 22, 25, 29, 32, 36, 39, 43 et 46 et la gemcitabine est administrée aux jours 4, 11, 18, 25, 32, 39 et 46 d'un premier cycle de traitement de sept semaines, en particulier dans laquelle le premier cycle de traitement est suivi d'un ou plusieurs cycles de traitement ultérieurs, la formulation liposomale cationique est administrée aux jours 1, 4, 8, 11, 15 et 18 et la gemcitabine est administrée aux jours 4, 11 et 18 d'un cycle de traitement ultérieur de trois semaines, et un intervalle de dosage entre un premier cycle de traitement et un cycle de traitement ultérieur ou entre deux cycles de traitement ultérieurs est d'une semaine.

**8.** La formulation liposomale cationique pour une utilisation selon l'une quelconque des revendications 1(a) et 2 à 4, dans laquelle la formulation liposomale cationique comprend :

(i) un lipide cationique de 30 % en moles à 99,9 % en moles, du paclitaxel en une quantité d'au moins 0,1 % en moles et un lipide neutre ou anionique en une quantité de 30 % en moles à 55 % en moles, et la formulation liposomale cationique a un potentiel zêta positif dans une solution de KCl à 0,05 M à pH 7,5 à température ambiante,
(ii) du DOTAP, du DOPC et du paclitaxel, ou
(iii) du DOTAP, du DOPC et du paclitaxel dans un rapport molaire de 50:47:3, ou
(iv) dans laquelle le lipide cationique est le sel de N-[1-(2,3-dioléoyloxy)propyl]-N,N,N-triméthylammonium (DOTAP) ; le bromure de diméthyldioctadécylammonium (DDAB) ; le propane 1,2-diacyloxy-3-triméthylammonium ; l'amine N-[1-(2,3-dioléoyloxy)propyl]-N,N-diméthylique (DODAP) ; le propane 1,2-diacyloxy-3-diméthylammonium ; le chlorure de N-[1-(2,3-dioléyloxy)propyl]-N,N,N-triméthylammonium (DOTMA) ; le propane 1,2-dialkyloxy-3-diméthylammonium ; la dioctadécylamidoglycylspermine (DOGS) ; le 3β-[N-(N',N'-diméthylaminoéthane)carbamoyl]cholestérol (DC-Chol) ; le trifluoroacétate de 2,3-dioléoyloxy-N-(2-(sperminecarboxamido)-éthyl)-N,N-diméthyl-1-propanaminium (DOSPA) ; le β-alanyl cholestérol ; le bromure de cétyltriméthylammonium (CTAB) ; la diC14-amidine ; la N-tert-butyl-N'-tétradécyl-3-tétradécylamino-propionamidine ; le 14Dea2 ; le chlorure de N-(alpha-triméthylammonioacétyl)didodécyl-D-glutamate (TMAG) ; le chlorure de O,O'-ditétradécanoyl-N-(triméthylammonioacétyl)diéthanolamine ; le 1,3-dioléoyloxy-2-(6-carboxy-spermyl)-propylamide (DOSPER) ; l'iodure de N,N,N',N'-tétraméthyl-N,N'-bis(2-hydroxyéthyl)-2,3-dioléoyloxy-1,4-butanediammonium ; le chlorure de 1-[2-(acyloxy)éthyl]-2-alkyl(alcényl)-3-(2-hydroxyéthyl)-imidazolinium ; le bromure de 1,2-dioléoyl-3-diméthyl-hydroxyéthylammonium (DORI) ; le bromure de 1,2-dioléyloxypropyl-3-diméthylhydroxyéthylammonium (DORIE) ; le bromure de 1,2-dioléyloxypropyl-3-diméthylhydroxypropylammonium (DORIE-HP) ; le bromure de 1,2-dioléyloxypropyl-3-diméthylhydroxybutylammonium (DORIE-HS) ; le bromure de 1,2-dioléyloxypropyl-3-diméthylhydroxypentylammonium (DORIE-Hpe) ; le bromure de 1,2-dimyristyloxypropyl-3-diméthylhydroxyéthylammonium (DMRIE) ; le bromure de 1,2-dipalmityloxypropyl-3-diméthylhydroxyéthylammonium (DPRIE) ; le bromure de 1,2-distéaryloxypropyl-3-diméthylhydroxyéthylammonium (DSRIE) ; ou la 1,2-diacyl-sn-glycérol-3-éthylphosphocholine, en particulier dans laquelle le chlorure de 1-[2-(acyloxy)éthyl]-2-alkyl(alcényl)-3-(2-hydroxyéthyl)-imidazolinium est le chlorure de 1-[2-(9(Z)-octadécénoyloxy) éthyl]-2-(8(Z)-heptadécényl-3-(2-hydroxyéthyl)-imidazolinium (DOTIM) ou le chlorure de 1-[2-(hexadécanoyloxy)éthyl]-2-pentadécyl-3-(2-hydroxyéthyl)imidazolinium (DPTIM).

**9.** La formulation liposomale cationique pour une utilisation selon la revendication 8(i), dans laquelle le lipide neutre est :

(a) le cholestérol, un phospholipide, un lysolipide, un sphingolipide ou un lipide pégylé avec une charge neutre,
(b) un lysophospholipide, ou

(c) la 1,2-diacyl-sn-glycéro-3-phosphoéthanolamine, la 1,2-diacyl-sn-glycéro-3-phosphocholine ou la sphingomyéline, en particulier dans laquelle la 1,2-diacyl-sn-glycéro-3-phosphoéthanolamine est la 1,2-dioléoyl-sn-glycéro-3-phosphoéthanolamine (DOPE) ou la 1,2-dioléoyl-sn-glycéro-3-phosphocholine (DOPC).

**10.** La formulation liposomale cationique pour une utilisation selon la revendication 2, dans laquelle la formulation liposomale cationique et la gemcitabine sont administrées simultanément mais séparément ou sont administrées séquentiellement.

**11.** La formulation liposomale cationique pour une utilisation selon la revendication 1(b), dans laquelle la méthode comprend en outre l'administration d'une quantité thérapeutiquement efficace de gemcitabine, en particulier dans laquelle la formulation liposomale cationique et la quantité thérapeutiquement efficace de gemcitabine sont administrées simultanément, mais séparément, ou sont administrées séquentiellement.

**12.** La formulation liposomale cationique pour une utilisation selon la revendication 1(a) ou 2, dans laquelle la méthode est précédée d'une thérapie néoadjuvante.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005039533 A1
- WO 2006117220 A2
- WO 9918113 A
- WO 9914209 A
- WO 9909021 A
- WO 9822451 A
- US 5869680 A
- WO 9828288 A
- US 5821263 A
- US 5415869 A
- WO 9407882 A
- WO 9407881 A
- WO 9407880 A
- WO 9407876 A
- WO 9323555 A
- WO 9310076 A
- US 5294637 A
- US 5283253 A
- US 5279949 A
- US 5274137 A
- US 5202448 A
- US 5200534 A
- US 5229529 A
- EP 590267 A
- WO 2004002468 A


### Non-patent literature cited in the description


- *Cancer Res.*, 2014, vol. 74, 2913-21
- **BURRIS et al.** *J Clin Oncol.*, 1997, vol. 15 (6), 2403-13
- *J Clin Oncol.*, 2007, vol. 25 (15), 1960-66
- *J Clin Oncol.*, 2013, vol. 31 (13), 1640-8
- *N Engl J Med*, 2011, vol. 364, 1817-25
- *N Engl J Med*, 2013, vol. 369, 1691-703
- *PLoS One.*, 04 November 2015, vol. 10, 11
- **KO et al.** *British Journal of Cancer*, 2013, vol. 109, 920-925
- **EICHHORN et al.** *Int. J. of Cancer*, 2010, vol. 126 (5), 1235-1245
- **XU et al.** *Practical Pharmacy and Clinical Remedies*, 2016, vol. 19 (4), 510-517
- **LÖHR et al.** *Annals of Oncology*, 2012, vol. 23 (5), 1214-1222
- **FENG YANG et al.** *Cancer Treatment reviews*, 2011, vol. 37 (8), 633-642